# EUROPÄISCHE PATENTSCHRIFT

(11) **EP 0 719 261 B1**
(45) Veröffentlichungstag und Bekanntmachung des Hinweises auf die Patenterteilung: **05.12.2001**
(21) Anmeldenummer: 94927589.5
(22) Anmeldetag: 08.09.1994
(51) Int. Cl.: C07D 261/04, C07D 413/04, A01N 25/32

(54) **SUBSTITUIERTE ISOXAZOLINE, VERFAHREN ZU DEREN HERSTELLUNG, DIESE ENTHALTENDE MITTEL UND DEREN VERWENDUNG ALS SAFENER**
SUBSTITUTED ISOXAZOLINES, PROCESS FOR PRODUCING THEM, AGENTS CONTAINING THEM AND THEIR USE AS SAFENERS
ISOXAZOLINES SUBSTITUEES, LEUR PROCEDE DE PREPARATION, AGENTS LES CONTENANT ET LEUR UTILISATION COMME REDUCTEURS DE PHYTOTOXICITE

(30) Priorität: 16.09.1993 DE 4331448
(43) Veröffentlichungstag der Anmeldung: 03.07.1996
(73) Patentinhaber: Aventis CropScience GmbH, 65929 Frankfurt am Main (DE)
(72) Erfinder: WILLMS, Lothar, D-65719 Hofheim (DE); BAUER, Klaus, D-63456 Hanau (DE); BIERINGER, Hermann, D-65817 Eppstein/Taunus (DE)
(86) Internationale Anmeldenummer: EP9403008
(87) Internationale Veröffentlichungsnummer: WO9507897

(56) Entgegenhaltungen:
- EP-A- 0 509 433
- WO-A-91/08202
- JOURNAL OF INDIAN CHEMICAL SOCIETY, Bd.70, Nr.2, Februar 1993, CALCUTTA Seiten 134 - 137 A. NAGARAJAN ET AL 'Electron-impact mass spectral fragmentation patterns of isoxazolines'
- CHEMICAL ABSTRACTS, vol. 67, no. 21, 20. November 1967, Columbus, Ohio, US; abstract no. 100039w, V.A. TARTAKOVSKII ET AL '1-3 dipolar cycloaddition of nitrone esters to conjugated unsaturated compounds' Seite 9407 ; & ZH. ORG. KHIM., Bd.3, Nr.6, 1967 Seiten 980 - 983
- CHEMICAL ABSTRACTS, vol. 120, no. 11, 14. März 1994, Columbus, Ohio, US; abstract no. 134457j, Seite 1040 ; & JP,A,5 213 909 (YOSHITOMI PHARMACEUTICAL) 24. August 1993
- ANGEWANDTE CHEMIE. INTERNATIONAL EDITION, Bd.18, Nr.1, 1979, WEINHEIM DE Seiten 78 - 79 VOLKER JÄGER ET AL 'Ring-opening of 5-(bromomethyl)-2-isoxazolines to beta,gamma-enoximes.'

## Beschreibung

Die Erfindung betrifft das technische Gebiet der Pflanzenschutzmittel, insbesondere Wirkstoff-Antidot-Kombinationen, die hervorragend für den Einsatz gegen konkurrierende Schadpflanzen in Nutzpflanzenkulturen geeignet sind.

Bei der Anwendung von Pflanzenbehandlungsmitteln, insbesondere bei der Anwendung von Herbiziden, können unerwünschte Schäden an den behandelten Kulturpflanzen auftreten. Viele Herbizide sind nicht voll verträglich (selektiv) mit einigen wichtigen Kulturpflanzen, so daß ihrem Einsatz enge Grenzen gesetzt sind. Sie können deshalb manchmal überhaupt nicht oder nur in solch geringen Aufwandmengen eingesetzt werden, daß die erwünschte breite herbizide Wirksamkeit gegen die Schadpflanzen nicht gewährleistet ist. So können beispielsweise viele Herbizide der weiter unten genannten Stoffklassen (A) bis (K) nicht ausreichend selektiv in Mais, Reis oder in Getreide eingesetzt werden. Besonders bei der Nachauflaufapplikation dieser Herbizide treten phytotoxische Nebenwirkungen an den Kulturpflanzen auf, und es ist wünschenswert, eine derartige Phytotoxizität zu vermeiden oder zu verringern.

Es ist bereits bekannt, Herbizide in Kombination mit Verbindungen einzusetzen, welche die Phytotoxizität der Herbizide bei Kulturpflanzen reduzieren, ohne die herbizide Wirkung gegen die Schadpflanzen entsprechend zu reduzieren. Solche Kombinationspartner werden "Safener" oder "Antidots" genannt.

Aus EP-A-509 433 (CA-A-2065983) ist die Verwendung von 5-Phenylisoxazolin- und 5-Phenylisothiazolin-3-carboxylderivaten als Safener für Herbizide aus der Reihe der Carbamate, Thiocarbamate, Halogenacetanilide, Phenoxyphenoxy-alkancarbonsäurederivate, Sulfonylharnstoffe etc. bekannt.

In EP-A-520371 (CA-A-2072229) werden u.a. 5-Alkylisoxazolin- und-isothiazolin-3-carboxylderivate als Safener für verschiedene Herbizidklassen genannt.

WO 92/03053 (CA-A-2089651) beschreibt die Verwendung von substituierten 3-Aryl-isoxazolin- und -isothiazolin-5-carboxylderivaten als Safener für diese Herbizide. In WO 91/18907 (US-A-5,332,715) werden silylsubstituierte Isoxazoline, Isoxazole, Isothiazoline und Isothiazole als pflanzenschützende Mittel beschrieben.

WO 91/08202 (US-A-5,314,863) schließlich beschreibt 5-benzyl-substitutierte Isoxazolinderivate mit pflanzenschützenden Eigenschaften.

Es wurde nun gefunden, daß sich überraschenderweise Verbindungen aus der Gruppe von 5,5-disubstituierten Isoxazolinderivaten der nachstehenden Formel (I) hervorragend dazu eignen, Kulturpflanzen gegen schädigende Wirkungen von aggressiven Agrarchemikalien, insbesondere Herbiziden, zu schützen.

Diese Isoxazoline, welche zum Schützen von Kulturpflanzen gegen schädigende Wirkungen von aggressiven Agrarchemikalien geeignet sind, entsprechen der Formel (I), 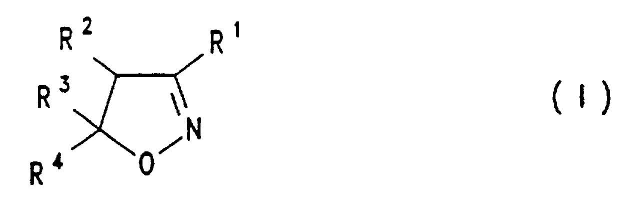
- R¹: worin einen Acylrest aus der Gruppe der Reste der Formel 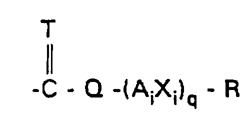 und 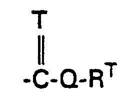 worin R, R^{T}, Q, Aᵢ, Xᵢ und q wie weiter unten definiert sind,
- R²: Wasserstoff
- R³ und R⁴: unabhängig voneinander C₁-C₁₈-Alkyl, C₃-C₁₂-Cycloalkyl, C₂-C₈-Alkenyl oder C₂-C₈-Alkinyl, oder einen Rest der Formel 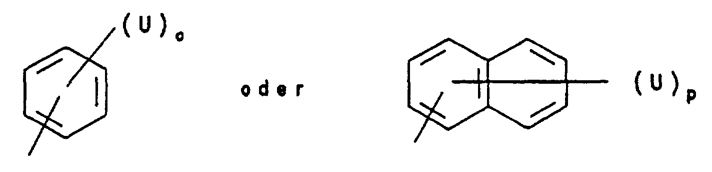 worin
(U) für gleiche oder verschiedene Reste stehen, welche unabhängig voneinander Wasserstoff, Halogen, Cyano, Nitro, Amino oder C₁-C₈-Haloalkyl, C₁-C₈-Haloalkoxy, C₁-C₈-Alkyl, C₁-C₈-Alkoxy, Mono-(C₁-C₄-alkyl)-amino, Di-(C₁-C₄-alkyl)-amino, C₁-C₈-Alkylthio oder C₁-C₈-Alkylsulfonyl, wobei jeder der letztgenannten 8 Reste unsubstituiert oder durch einen oder mehrere, vorzugsweise bis zu drei gleiche oder verschiedene Substituenten aus der Gruppe enthaltend Halogen, C₁-C₈-Haloalkoxy, Nitro, Cyano, Hydroxy, C₁-C₈-Alkoxy und eine C₁-C₈-Alkoxygruppe, worin eine oder mehrere, vorzugsweise bis zu drei CH₂-Gruppen durch Sauerstoff ersetzt sind, und C₁-C₈-Alkylthio, C₁-C₆-Alkylsulfinyl, C₁-C₆-Alkylsulfonyl, C₂-C₈-Alkenylthio, C₂-C₈-Alkinylthio, C₂-C₈-Alkenyloxy, C₂-C₈-Alkinyloxy, C₃-C₇-Cycloalkyl, C₃-C₇-Cycloalkoxy, Mono- und Di-(C₁-C₄-alkyl)-amino und (C₁-C₈-Alkoxy)-carbonyl substituiert ist, und vorzugsweise Wasserstoff, Halogen, C₁-C₆-Haloalkyl, wie Trifluormethyl, C₁-C₆-Haloalkoxy, wie Difluormethoxy, C₁-C₆-Alkyl, C₁-C₆-Alkoxy, C₁-C₆-Alkylthio, C₁-C₆-Alkylsulfonyl, Nitro, Amino, (C₁-C₂-Alkyl)-amino, Di-(C₁-C₂-alkyl)-amino oder Cyano bedeuten und
O eine ganze Zahl von 1 bis 5, vorzugsweise 1 bis 3, ist und
p eine ganze Zahl von 1 bis 7, vorzugsweise 1 bis 3, ist, oder vorzugsweise einen monocyclischen oder bicyclischen Heteroarylrest aus der Gruppe Furyl, Thienyl, Pyrrolyl, Pyrazolyl, Thiazolyl, Oxazolyl, Pyridinyl, Pyrimidinyl, Pyrazinyl, Pyridazinyl und Chinolinyl, der jeweils unsubstituiert oder durch einen oder mehrere, vorzugsweise ein bis drei der genannten Reste U substituiert ist,
- R: Wasserstoff oder einen aliphatischen, aromatischen, heteroaromatischen, araliphatischen oder heteroaraliphatischen Rest mit 1 bis 30 C-Atomen, der unsubstituiert oder mit einer oder mehreren funktionellen Gruppen substituiert ist, insbesondere R einen Rest Wasserstoff, C₁-C₁₈-Alkyl, C₃-C₁₂-Cycloalkyl, C₂-C₈-Alkenyl oder C₂-C₈-Alkinyl, Heterocyclyl, Phenyl oder Heteroaryl,
wobei jeder der letztgenannten 7 Reste unabhängig voneinander unsubstituiert oder durch einen oder mehrere Reste aus der Gruppe enthaltend Halogen, Cyano, Thio, Nitro, Hydroxy, C₁-C₈-Alkyl, letzteres nur für den Fall cyclischer Reste, C₁-C₈-Haloalkyl, C₁-C₈-Alkoxy, C₂-C₈-Alkenyloxy, C₂-C₈-Alkinyloxy, C₁-C₈-Haloalkoxy, C₁-C₈-Alkylthio, C₂-C₈-Alkenylthio, C₂-C₈-Alkinylthio, C₃-C₇-Cycloalkyl, C₃-C₇-Cycloalkoxy, Reste der Formeln -NR*R** und -CO-NR*R** und -O-CO-NR*R**,
   wobei R* und R** in den letztgenannten 3 Resten unabhängig voneinander Wasserstoff, C₁-C₈-Alkyl, C₂-C₈-Alkenyl, C₂-C₈-Alkinyl, Benzyl, Phenyl oder substituiertes Phenyl sind oder gemeinsam mit dem N-Atom einen 3- bis 8-gliedrigen Heterocyclus, der noch bis zu 2 weitere Heteroatome aus der Gruppe N, O und S enthalten und durch C₁-C₄-Alkyl substituiert sein kann, bedeuten,
sowie (C₁-C₈-Alkoxy)-carbonyl, (C₁-C₈-Alkoxy)-thiocarbonyl, (C₂-C₈-Alkenyloxy)-carbonyl, (C₁-C₈-Alkylthio)-carbonyl, (C₂-C₈-Alkenylthio)-carbonyl, (C₂-C₈-Alkinylthio)-carbonyl, (C₂-C₈-Alkinyloxy)-carbonyl, Formyl, (C₁-C₈-Alkyl)-carbonyl, (C₂-C₈-Alkenyl)-carbonyl, (C₂-C₈-Alkinyl)-carbonyl, C₁-C₄-Alkylimino, C₁-C₄-Alkoxyimino, (C₁-C₈-Alkyl)-carbonylamino, (C₂-C₈-Alkenyl)-carbonylamino, (C₂-C₈-Alkinyl)-carbonylamino, (C₁-C₈-Alkoxy)-carbonylamino, (C₂-C₈-Alkenyloxy)-carbonylamino, (C₂-C₈-Alkinyloxy)-carbonylamino, (C₁-C₈-Alkyl)-amino-carbonylamino, (C₁-C₆-Alkyl)-carbonyloxy, das unsubstituiert oder durch Halogen, NO₂, C₁-C₄-Alkoxy oder gegebenenfalls substituiertes Phenyl substituiert ist, und (C₂-C₆-Alkenyl)-carbonyloxy, (C₂-C₆-Alkinyl)-carbonyloxy, (C₁-C₈-Alkoxy)-carbonyloxy, (C₂-C₈-Alkenyloxy)-carbonyloxy, (C₂-C₈-Alkinyloxy)-carbonyloxy, C₁-C₈-Alkylsulfonyl, Phenyl, Phenyl-C₁-C₆-alkoxy, Phenyl-(C₁-C₆-alkoxy)-carbonyl, Phenoxy, Phenoxy-C₁-C₆-alkoxy, Phenoxy-(C₁-C₆-alkoxy)-carbonyl, Phenoxycarbonyl, Phenylcarbonyloxy, Phenylcarbonylamino, Phenyl-(C₁-C₆-alkyl)-carbonylamino und Phenyl-(C₁-C₆-alkyl)-carbonyloxy,
   wobei die letztgenannten 11 Reste im Phenylring unsubstituiert oder durch einen oder mehrere Reste aus der Gruppe Halogen, C₁-C₄-Alkyl, C₁-C₄-Alkoxy, C₁-C₄-Haloalkyl, C₁-C₄-Haloalkoxy und Nitro substituiert sind,
   und Reste der Formeln -SiR'₃, -O-SiR'₃, (R')₃Si-C₁-C₆-alkoxy, -CO-O-NR'₂, -O-N =CR'₂, -N=CR'₂, -O-NR'₂, -CH(OR')₂ und -O-(CH₂)ₘ-CH(OR')₂,
   worin die R' in den genannten Formeln unabhängig voneinander Wasserstoff, C₁-C₄-Alkyl oder Phenyl, das unsubstituiert oder ein- oder mehrfach durch Reste aus der Gruppe Halogen, C₁-C₄-Alkyl, C₁-C₄-Alkoxy, C₁-C₄-Haloalkyl, C₁-C₄-Haloalkoxy und Nitro substituiert ist, oder paarweise eine C₂-C₆-Alkylenkette und m = 0 bis 6 bedeuten,
und einen substituierten Alkoxyrest der Formel R"O-CHR"'CH(OR")-C₁-C₆-alkoxy,
   worin die R" unabhängig voneinander C₁-C₄-Alkyl oder zusammen eine C₁-C₆-Alkylengruppe und R"' Wasserstoff oder C₁-C₄-Alkyl bedeuten,
substituiert ist,
- R^{T}: einen Rest der Formel -CO-R, -CS-R, -NR^{f}R^{g}, -N=CR^{h}Rⁱ oder SiR^{a}R^{b}R^{c},
wobei R die genannte Bedeutung hat und R^{f}, R^{g}, R^{h} und Rⁱ unabhängig voneinander Wasserstoff, C₁-C₄-Alkyl, C₂-C₄-Alkenyl, C₂-C₄-Alkinyl, Benzyl, Phenyl oder substituiertes Phenyl sind oder R^{f} und R^{g} gemeinsam mit dem N-Atom einen 5- oder 6-gliedrigen Heterocyclus, der noch bis zu 2 weitere Heteroatome aus der Gruppe N, O und S enthalten und durch C₁-C₄-Alkyl substituiert sein kann, bedeuten und R^{a}, R^{b} und R^{c} unabhängig voneinander C₁-C₄-Alkyl, C₂-C₄-Alkenyl, C₂-C₄-Alkinyl, Phenyl oder substituiertes Phenyl sind,
- T: O, S, NR⁸, N-OR⁸ oder N-O-Acyl,
- Q: O oder S,
- q: eine ganze Zahl von O bis 4,
- i: eine Laufziffer, welche bei q ungleich 0 alle ganzen Zahlen von 1 bis q annimmt, wobei q die oben angegebene Bedeutung hat,
- Xᵢ: unabhängig voneinander O, S, NR⁹, N-(AᵢXᵢ)_{q}-R
- Aᵢ: unabhängig voneinander unsubstituiertes oder substituiertes C₁-C₆-Alkylen, C₂-C₆-Alkenylen, C₂-C₆-Alkinylen, C₃-C₆-Cycloalkylen, C₃-C₆-Cycloalkenylen, Heterocyclylen, Arylen oder Heteroarylen und
- R⁸, R⁹: unabhängig voneinander H, C₁-C₄-Alkyl, C₂-C₄-Alkenyl, C₂-C₄-Alkinyl, C₃-C₆-Cycloalkyl, C₃-C₆-Cycloalkenyl, Heterocyclyl, Aryl oder Heteroaryl
bedeuten.

In der Formel (I) und im folgenden können die Reste Alkyl, Alkoxy, Haloalkyl, Haloalkoxy, Alkylamino und Alkylthio sowie die entsprechenden ungesättigten und/oder substituierten Reste im Kohlenstoffgerüst jeweils geradkettig oder verzweigt sein. Wenn nicht speziell angegeben, sind bei diesen Resten die Kohlenstoffgerüste mit 1 bis 4 C-Atomen, bzw. bei ungesättigten Gruppen mit 2 bis 4 C-Atomen bevorzugt. Alkylreste, auch in den zusammengesetzten Bedeutungen wie Alkoxy, Haloalkyl usw., bedeuten z. B. Methyl, Ethyl, n- oder i-Propyl, n-, i-, t- oder 2-Butyl, Pentyle, Hexyle, wie n-Hexyl, i-Hexyl und 1,3-Dimethylbutyl, Heptyle, wie n-Heptyl, 1-Methylhexyl und 1,4-Dimethylpentyl; Alkenyl- und Alkinylreste haben die Bedeutung der den Alkylresten entsprechenden möglichen ungesättigten Reste, Alkenyl bedeutet z.B. Allyl, 1-Methylprop-2-en-1-yl, 2-Methyl-prop-2-en-1-yl, But-2-en-1-yl, But-3-en-1-yl, 1-Methyl-but-3-en-1-yl und 1-Methyl-but-2-en-1-yl; Alkinyl bedeutet z.B. Propargyl, But-2-in-1-yl, But-3-in-1-yl, 1-Methyl-but-3-in-1-yl.
Halogen bedeutet Fluor, Chlor, Brom oder lod, vorzugsweise Fluor, Chlor oder Brom, insbesondere Fluor oder Chlor. Haloalkyl, -alkenyl und -alkinyl bedeuten durch Halogen teilweise oder vollständig substituiertes Alkyl, Alkenyl bzw. Alkinyl, z.B. CF₃, CHF₂, CH₂F, CF₃CF₂, CH₂FCHCl, CCl₃, CHCl₂, CH₂CH₂Cl; Haloalkoxy ist z.B. OCF₃, OCHF₂, OCH₂F, CF₃CF₂O, OCH₂CF₃. Entsprechendes gilt für Haloalkenyl und andere durch Halogen substituierte Reste.

Aryl bedeutet beispielsweise Phenyl, Naphthyl, Tetrahydronaphthyl, Indenyl, Indanyl, Pentalenyl, Fluorenyl und ähnliches, insbesondere Phenyl; Aryloxy bedeutet vorzugsweise die den genannten Arylresten entsprechenden Oxy-Reste, insbesondere Phenoxy.

Heteroaryl bzw. Heteroaryl in Heteroaryloxy bedeutet insbesondere Pyridyl, Pyrimidinyl, Pyridazinyl, Pyrazinyl, Thienyl, Thiazolyl, Oxazolyl, Furyl, Pyrrolyl, Pyrazolyl und Imidazolyl, aber auch bicyclische oder polycyclische aromatische oder araliphatische Verbindungen, z. B. Chinolinyl, Benzoxazolyl etc.

Substituiertes Aryl bzw. Aryloxy, Heteroaryl, Heteroaryloxy, Phenyl, Phenoxy, Benzyl, Benzyloxy bzw. substituierte bicyclische Reste mit aromatischen Anteilen bedeuten beispielsweise einen vom unsubstituierten Grundkörper abgeleiteten substituierten Rest, wobei die Substituenten insbesondere ein oder mehrere, vorzugsweise 1, 2 oder 3 Reste aus der Gruppe Halogen, Alkyl, Haloalkyl, Alkoxy, Haloalkoxy, Hydroxy, Amino, Nitro, Cyano, Alkoxycarbonyl, Alkylcarbonyl, Formyl, Carbamoyl, Mono- und Dialkylaminocarbonyl, Mono- und Dialkylamino, Alkylsulfinyl und Alkylsulfonyl bedeuten und bei Resten mit C-Atomen solche mit 1 bis 4 C-Atomen, insbesondere 1 oder 2, bevorzugt sind. Bevorzugt sind dabei in der Regel Substituenten aus der Gruppe Halogen, z. B. Fluor und Chlor, C₁-C₄-Alkyl, vorzugsweise Methyl oder Ethyl, C₁-C₄-Haloalkyl, vorzugsweise Trifluormethyl, C₁-C₄-Alkoxy, vorzugsweise Methoxy oder Ethoxy, C₁-C₄-Haloalkoxy, Nitro und Cyano. Besonders bevorzugt sind dabei die Substituenten Methyl, Methoxy und Chlor.

Gegebenenfalls substituiertes Phenyl ist z.B. Phenyl, das unsubstituiert oder ein-oder mehrfach, vorzugsweise bis zu dreifach durch gleiche oder verschiedene Reste aus der Gruppe Halogen, (C₁-C₄)-Alkyl, (C₁-C₄)-Alkoxy, (C₁-C₄)-Halogenalkyl, (C₁-C₄)-Halogenalkoxy und Nitro substituiert ist, z.B. o-, m- und p-Tolyl, Dimethylphenyle, 2-, 3- und 4-Chlorphenyl, 2-, 3- und 4-Trifluor- und -Trichlorphenyl, 2,4-, 3,5-, 2,5- und 2,3-Dichlorphenyl, o-, m- und p-Methoxyphenyl.

Ein drei- bis siebengliedriger wie oben beschriebener heterocyclischer Rest ist insbesondere von Benzol abgeleitet, wovon mindestens ein CH durch N und/oder mindestens zwei benachbarte CH-Paare durch NH, S und/oder O ersetzt sind. Der Rest kann benzokondensiert sein. Er ist gegebenenfalls teilweise oder vollständig hydriert und wird dann auch als Heterocyclyl bezeichnet. Es kommen insbesondere Reste wie Oxiranyl, Pyrrolidyl, Piperidyl, Dioxolanyl, Pyrazolyl, Morpholyl, Furyl, Tetrahydrofuryl, Indolyl, Chinolinyl, Pyrimidyl, Azepinyl, Triazolyl, Thienyl und Oxazolyl in Frage.

Acyl bedeutet beispielsweise Formyl, Alkylcarbonyl wie (C₁-C₄-Alkyl)-carbonyl, Phenylcarbonyl, wobei der Phenylring substituiert sein kann, z. B. wie oben für Phenyl gezeigt, oder Alkyloxycarbonyl, Phenyloxycarbonyl, Benzyloxycarbonyl, Alkylsulfonyl und andere Reste von organischen Säuren.
Derivate von Carboxy sind typische Säurederivatereste wie z.B. Salze, Ester, Thioester, Amide, Thioamide, Ketosäuren, Amidine und Nitrile. Derivate von Formyl und Acyl sind vor allem carbonylanaloge Derivate, wie Acetale, Thioacetale, Thioketale, Imine, Thioformyl, Thioacyl etc.

Manche Verbindungen der Formel (I) enthalten ein oder mehrere asymmetrische C-Atome oder Doppelbindungen, die in der allgemeinen Formel (I) nicht gesondert angegeben sind. Die durch ihre spezifische Raumform definierten möglichen Stereoisomeren, wie Enantiomere, Diastereomere, E- und Z-lsomere sowie deren Gemische sind jedoch alle von der Formel (I) umfaßt.

Die Verbindungen der Formel (I), welche von Carbonsäuren abgeleitet sind, können Salze bilden, bei denen der Rest R durch ein Äquivalent eines für die Landwirtschaft geeigneten Kations ersetzt wird. Diese Salze sind beispielsweise Metall-, insbesondere Alkalisalze (Na,K) oder Erdalkalisalze, aber auch Ammoniumsalze oder Salze mit organischen Aminen sowie Salze, die als Kationen Sulfonium- oder Phosphoniumionen enthalten.

Als Salzbildner eignen sich besonders Metalle und organische Stickstoffbasen, vor allem quartäre Ammoniumbasen. Hierbei kommen als zur Salzbildung geeignete Metalle Erdalkalimetalle, wie Magnesium oder Calcium, vor allem aber Alkalimetalle in Betracht, wie Lithium und insbesondere Kalium und Natrium.

Beispiele für zur Salzbildung geeignete Stickstoffbasen sind primäre, sekundäre oder tertiäre, aliphatische und aromatische, gegebenenfalls am Kohlenwasserstoffrest hydroxylierte Amine, wie Methylamin, Ethylamin, Propylamin, Isopropylamin, die vier isomeren Butylamine, Dimethylamin, Diethylamin, Dipropylamin, Diisopropylamin, Di-n-butylamin, Pyrrolidin, Piperidin, Morpholin, Trimethylamin, Triethylamin, Tripropylamin, Chinuclidin, Pyridin, Chinolin, Isochinolin sowie Methanolamin, Ethanolamin, Propanolamin, Dimethanolamin, Diethanolamin oder Triethanolamin.

Beispiele für quartäre Ammoniumbasen sind Tetraalkylammoniumkationen, in denen die Alkylreste unabhängig voneinander geradkettige oder verzweigte C₁-C₆-Alkylgruppen sind, wie das Tetramethylammoniumkation, das Tetraethylammoniumkation oder das Trimethylethylammoniumkation, sowie weiterhin das Trimethylbenzylammoniumkation, das Triethylbenzylammoniumkation und das Trimethyl-2-hydroxyethylammoniumkation.

Besonders bevorzugt als Salzbildner sind das Ammoniumkation und Di-sowie Trialkylammoniumkationen, in denen die Alkylreste unabhängig voneinander geradkettige oder verzweigte, gegebenenfalls durch eine Hydroxylgruppe substituierte (C₁-C₆)-Alkylgruppen darstellen, wie beispielsweise das Dimethylammoniumkation, das Trimethylammoniumkation, das Triethylammoniumkation, das Di-(2-hydroxyethyl)-ammoniumkation und das Tri-(2-hydroxyethyl)-ammoniumkation.

Von besonderem Interesse sind Verbindungen der Formel (I) oder deren Salze,
worin
- R²: Wasserstoff und mindestens einer der Reste R³ und R⁴ einen Rest der Formel 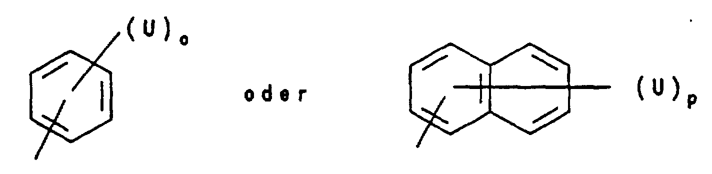 worin
(U) für gleiche oder verschiedene Reste stehen, welche unabhängig voneinander Wasserstoff, Halogen, wie Fluor, Chlor, Brom und lod, Cyano, Nitro, Amino, C₁-C₄-Haloalkyl, C₁-C₄-Haloalkoxy, C₁-C₄-Alkyl, C₁-C₄-Alkoxy, Mono-(C₁-C₄-alkyl)-amino, Di-(C₁-C₄-alkyl)-amino, C₁-C₄-Alkylthio oder C₁-C₄-Alkylsulfonyl bedeuten,
O eine ganze Zahl von 1 bis 3 ist und
p eine ganze Zahl von 1 bis 3 ist, oder
- R³, R⁴: unabhängig voneinander einen monocyclischen oder bicyclischen Heteroarylrest aus der Gruppe Furyl, Thienyl, Pyrrolyl, Pyrazolyl, Thiazolyl, Oxazolyl, Pyridinyl, Pyrimidinyl, Pyrazinyl, Pyridazinyl und Chinolinyl, der unsubstituiert oder durch ein bis drei der vorstehend genannten Reste U substituiert ist, bedeuten.

Besonders bevorzugt sind die Reste R³ und R⁴ gleiche oder verschiedene Reste der Formel 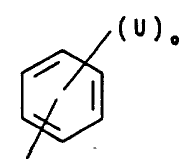 wobei U und o die vorstehend genannten Bedeutungen haben.

Von besonderem Interesse sind auch Verbindungen der genannten Formel (I) und deren Salze, worin
- R: Wasserstoff, C₁-C₈-Alkyl, C₄-C₇-Cycloalkyl, C₂-C₈-Alkenyl oder C₂-C₈-Alkinyl, Heterocyclyl, Phenyl oder Heteroaryl ist,
wobei jeder der letztgenannten 7 Reste unabhängig voneinander unsubstituiert oder durch einen oder mehrere Reste aus der Gruppe enthaltend Halogen, Cyano, Thio, Nitro, Hydroxy, C₁-C₄-Alkyl, letzteres nur für den Fall cyclischer Reste, C₁-C₄-Haloalkyl, C₁-C₄-Alkoxy, C₂-C₄-Alkenyloxy, C₂-C₄-Alkinyloxy, C₁-C₄-Haloalkoxy, C₁-C₄-Alkylthio, C₂-C₄-Alkenylthio, C₂-C₄-Alkinylthio, C₅-C₆-Cycloalkyl, C₅-C₆-Cycloalkoxy, Amino, Mono- und Di-(C₁-C₄-alkyl)-amino, (C₁-C₆-Alkoxy)-carbonyl, Reste der Formeln -SiR'₃, -O-NR'₂, -O-N=CR'₂, -N=CR'₂, worin die R' in den genannten Formeln unabhängig voneinander Wasserstoff, C₁-C₂-Alkyl oder Phenyl oder paarweise eine C₂-C₅-Alkylenkette bedeuten, substituiert ist,
bedeutet, oder
Verbindungen, worin
- R^{T}: einen Rest der Formel -CO-R, -NR^{f}R^{g} oder -N =CR^{h}Rⁱ, wobei R, R^{f}, R^{g}, R^{h} und R' die genannten Bedeutungen haben, bedeutet.

Vorzugsweise bedeutet R Wasserstoff, C₁-C₈-Alkyl, C₅-C₆-Cycloalkyl, C₂-C₈-Alkenyl oder C₂-C₈-Alkinyl, wobei jeder der letztgenannten 4 Reste unabhängig voneinander unsubstituiert oder durch einen oder mehrere Reste aus der Gruppe enthaltend Halogen, Cyano, Nitro, C₁-C₄-Alkoxy, C₂-C₄-Alkenyloxy, C₂-C₄-Alkinyloxy, C₅-C₆-Cycloalkyl, C₅-C₆-Cycloalkoxyl Mono- und Di-(C₁-C₄-alkyl)-amino, Reste der Formeln -SiR'₃, -O-N=CR'₂, -N=CR'₂, worin die R' in den genannten Formeln unabhängig voneinander Wasserstoff, C₁-C₂-Alkyl oder Phenyl oder paarweise eine C₂-C₅-Alkylenkette bedeuten, substituiert ist.
- R^{T}: bedeutet vorzugsweise -CO-R, wobei R die genannte Bedeutung hat, oder -NR^{f}R^{g} oder -N=CR^{h}Rⁱ, worin
- R^{f}, R^{g}: unabhängig voneinander H, C₁-C₂-Alkyl, Benzyl oder Phenyl oder gemeinsam mit dem N-Atom Pyrrolidin-1-yl, Piperidin-1-yl, Morpholin-4-yl, Piperazin-1-yl oder Imidazol-1-yl, bzw.
- R^{h}, Rⁱ: unabhängig voneinander H, C₁-C₂-Alkyl, Benzyl oder Phenyl bedeuten.

Von besonderem Interesse sind auch Verbindungen der genannten Formel (I) und deren Salze, worin
- T: O, S oder NR⁸, vorzugsweise O oder NR⁸,
- Q: O oder S, vorzugsweise O,
- q: eine ganze Zahl von 0 bis 4,
- i: eine Laufziffer, welche bei q ungleich 0 alle ganzen Zahlen von 1 bis q annimmt, wobei q die oben angegebene Bedeutung hat,
- Xᵢ: unabhängig voneinander O, S, NR⁹, N-(AᵢXᵢ)_{q}-R
- Aᵢ: unabhängig voneinander unsubstituiertes oder substituiertes C₁-C₄-Alkylen, C₂-C₄-Alkenylen, C₅-C₆-Cycloalkylen, vorzugsweise C₁-C₄-Alkylen,
- R⁸, R⁹: unabhängig voneinander H, C₁-C₄-Alkyl, C₂-C₄-Alkenyl, C₂-C₄-Alkinyl oder C₅-C₆-Cycloalkyl
bedeuten.

Die Erfindung betrifft auch ein Verfahren zum Schutz von Kulturpflanzen, vorzugsweise Getreide-, Reis-, Mais-, Sojabohnen- oder Zuckerrübenpflanzen, vor phytotoxischen Nebenwirkungen von Pflanzenschutzmitteln wie Herbiziden, Insektiziden und Fungiziden, das dadurch gekennzeichnet ist, daß eine wirksame Menge mindestens einer Verbindung der Formel (I) bzw. deren Salz vor, nach oder gleichzeitig mit den jeweiligen Wirkstoffen auf die Pflanzen, Pflanzensamen oder die Anbaufläche appliziert wird.

Die Erfindung betrifft weiterhin die Verwendung von Verbindungen der Formel (I) oder deren Salzen zum Schutz von Kulturpflanzen vor phytotoxischen Nebenwirkungen von Pflanzenschutzmitteln wie Herbiziden, Insektiziden und Fungiziden.

Gegenstand der vorliegenden Erfindung ist auch ein Verfahren zur Herstellung der Verbindung der Formel (I) und deren Salze, dadurch gekennzeichnet, daß man eine Verbindung der Formel (II)

R³R⁴C = CHR² (II)

worin R², R³ und R⁴ die in Formel (I) angegebene Bedeutung haben, mit einem Nitriloxid der Formel (III)

⁽⁻⁾O - N = ⁽⁺⁾C - R¹ (III)

worin R¹ die in Formel (I) angegebene Bedeutung hat, umsetzt.

Die Umsetzung wird beispielsweise in einem organischen Lösungsmittel durchgeführt. Als Lösungsmittel eignen sich vorzugsweise unpolare bis wenig polare organische Lösungsmittel, z. B. Ether wie Diethylether oder Tetrahydrofuran (THF).

Die Ausgangsverbindungen der Formel (II) und (III) sind literaturbekannt (vgl. J. Org. Chem. 25, 1160 (1960); J. Am. Chem. Soc. 46, 791 (1924) und dort genannte Zitate) oder lassen sich analog den bekannten Verbindungen herstellen. Die Nitriloxide der Formel (III) werden in der Regel in situ aus 2-Halogeno-2-hydroximinoessigsäure(derivaten) bzw. -ethanal(derivaten) bzw. -ketonen unter Einwirkung von Basen, z.B. organische Aminbasen, hergestellt und direkt mit schon in der Reaktionsmischung enthaltender Verbindung der Formel (II) umgesetzt. Die Umsetzung wird vorzugsweise bei einer Temperatur von -15°C bis zur Siedetemperatur des Lösungsmittels, insbesondere bei Raumtemperatur, durchgeführt.

Im folgenden sind mit Verbindungen der Formel (I) auch deren Salze eingeschlossen, sofern keine genauere Definition gegeben ist.

Verbindungen der Formel (I) reduzieren oder unterbinden phytotoxische Nebenwirkungen von Pflanzenschutzmitteln wie Herbiziden, Insektiziden und Fungiziden, die beim Einsatz dieser Wirkstoffe in Nutzpflanzenkulturen auftreten, und können deshalb in üblicher Weise als Antidote oder Safener bezeichnet werden.

Die erfindungsgemäßen Verbindungen der Formel (I) können zur gemeinsamen Anwendung mit Pflanzenschutzmittel-Wirkstoffen gleichzeitig oder in beliebiger Reihenfolge mit den Wirkstoffen ausgebracht werden und sind dann in der Lage, schädliche Nebenwirkungen dieser Wirkstoffe bei Kulturpflanzen zu reduzieren oder völlig aufzuheben, ohne die Wirksamkeit dieser Wirkstoffe gegen Schadpflanzen bzw. Schadinsekten oder Schadpilze zu beeinträchtigen. Dabei können auch Schädigungen, welche durch die Anwendung mehrerer Pflanzenschutzmittel entstehen, z. B. durch mehrere Herbizide oder durch Herbizide in Kombination mit Insektiziden oder Fungiziden, wesentlich reduziert oder völlig aufgehoben werden. Hierdurch kann das Einsatzgebiet herkömmlicher Pflanzenschutzmittel ganz erheblich erweitert werden.

Insektizide, die allein oder gemeinsam mit Herbiziden Pflanzenschädigungen verursachen können, sind beispielsweise folgende:
Insektizide Präparate wie Organophosphate z. B. Terbufos (® Counter), Fonofos (® Dyfonate), Phorate (® Thimet), Chlorpyrifos (® Reldan) und andere verwandte Wirkstoffe; insektizide Carbamate wie z. B. Carbofuran (® Furadan) und andere; sowie Pyrethroid-Insektizide wie z. B. Tefluthrin (® Force), Deltamethrin (® Decis) und Tralomethrin (® Scout) und andere; sowie andere insektizide Mittel mit andersartigem Wirkungsmechanismus.

Herbizide, deren phytotoxische Nebenwirkungen auf Kulturpflanzen mittels Verbindungen der Formel (I) herabgesetzt werden können, sind z.B. Herbizide aus der Gruppe der Carbamate, Thiocarbamate, Halogenacetanilide, substituierte Phenoxy-, Naphthoxy- und Phenoxy-phenoxycarbonsäurederivate sowie Heteroaryloxy-phenoxyalkancarbonsäurederivate, wie Chinolyloxy-, Chinoxalyloxy-, Pyridyloxy-, Benzoxalyloxy- und Benzthiazolyloxyphenoxyalkancarbonsäureester, Cyclohexandionabkömmlinge, Imidazolinone, Pyrimidyloxy-pyridincarbonsäure-derivate, Pyrimidyloxy-benzoesäure-derivate, Sulfonylharnstoffe, Triazolopyrimidin-sulfonamid-derivate sowie S-(N-Aryl-N-alkylcarbamoylmethyl)-dithiophosphorsäureester. Bevorzugt sind dabei Phenoxyphenoxy- und Heteroaryloxy-phenoxycarbonsäureester und -salze, Sulfonylharnstoffe, Imidazolinone sowie Herbizide, die gemeinsam mit ALS-Hemmstoffen (Acetolactat-Synthase-Hemmstoffen) zur Erweiterung des Wirkungsspektrums eingesetzt werden, z.B. Bentazon, Cyanazin, Atrazin, Bromoxynil, Dicamba und andere Blattherbizide.

Geeignete Herbizide, die mit den erfindungsgemäßen Safenern kombiniert werden können, sind beispielsweise:
A) Herbizide vom Typ der Phenoxyphenoxy- und Heteroaryloxyphenoxycarbonsäure-(C₁-C₄)alkyl-, (C₂-C₄)alkenyl- und (C₃-C₄)alkinylester wie
   A1) Phenoxy-phenoxy- und Benzyloxy-phenoxy-carbonsäure-derivate, z.B.
      2-(4-(2,4-Dichlorphenoxy)-phenoxy)-propionsäuremethylester(Diclofop-methyl),
      2-(4-(4-Brom-2-chlorphenoxy)-phenoxy)-propionsäuremethylester (s. DE-A-2601548),
      2-(4-(4-Brom-2-fluorphenoxy)-phenoxy)-propionsäuremethylester (s. US-A-4808750),
      2-(4-(2-Chlor-4-trifluormethylphenoxy)-phenoxy)-propionsäuremethylester (s. DE-A-2433067),
      2-(4-(2-Fluor-4-trifluormethylphenoxy)-phenoxy)-propionsäuremethylester (s. US-A-4808750),
      2-(4-(2,4-Dichlorbenzyl)-phenoxy)propionsäuremethylester (s. DE-A-2417487),
      4-(4-(4-Trifluormethylphenoxy)-phenoxy)-pent-2-en-säureethylester,
      2-(4-(4-Trifluormethylphenoxy)-phenoxy)-propionsäuremethylester (s. DE-A-2433067),
   A2) "Einkernige" Heteroaryloxy-phenoxy-alkancarbonsäurederivate, z.B.
      2-(4-(3,5-Dichlorpyridyl-2-oxy)-phenoxy)-propionsäureethylester (s. EP-A-2925),
      2-(4-(3,5-Dichlorpyridyl-2-oxy)-phenoxy)-propionsäurepropargylester (EP-A-3114),
      2-(4-(3-Chlor-5-trifluormethyl-2-pyridyloxy)-phenoxy-propionsäure-methylester (s. EP-A-3890),
      2-(4-(3-Chlor-5-trifluormethyl-2-pyridyloxy)-phenoxy)-propionsäure-ethylester (s. EP-A-3890),
      2-(4-(5-Chlor-3-fluor-2-pyridyloxy)-phenoxy)-propionsäurepropargylester (EP-A-191736),
      2-(4-(5-Trifluormethyl-2-pyridyloxyl-phenoxy)-propionsäurebutylester (Fluazifopbutyl),
   A3) "Zweikernige" Heteroaryloxy-phenoxy-alkancarbonsäurederivate, z.B.
      2-(4-(6-Chlor-2-chinoxalyloxy)-phenoxy)-propionsäuremethylester und -ethylester (Quizalofop-methyl und -ethyl),
      2-(4-(6-Fluor-2-chinoxalyloxy)-phenoxy)-propionsäuremethylester (s. J. Pest. Sci. Vol. 10, 61 (1985)),
      2-(4-(6-Chlor-2-chinoxalyloxy)-phenoxy)-propionsäure und -2-isopropylidenaminooxyethylester (Propaquizafop u. Ester),
      2-(4-(6-Chlorbenzoxazol-2-yl-oxy)-phenoxy)-propionsäureethylester (Fenoxapropethyl), dessen D(+) Isomer (Fenoxaprop-P-ethyl) und
      2-(4-(6-Chlorbenzthiazol-2-yloxy)-phenoxypropionsäureethylester (s. DE-A-2640730),
      2-(4-(6-Chlorchinoxalyloxy)-phenoxy-propionsäure-tetrahydrofur-2-ylmethyl-ester (s. EP-A 323 727),
B) Herbizide aus der Sulfonylharnstoff-Reihe, wie z.B. Pyrimidin- oder Triazinylaminocarbonyl-[benzol-, pyridin-, pyrazol-, thiophen- und (alkylsulfonyl)alkylamino-]-sulfamide. Bevorzugt als Substituenten am Pyrimidinring oder Triazinring sind Alkoxy, Alkyl, Haloalkoxy, Haloalkyl, Halogen oder Dimethylamino, wobei alle Substituenten unabhängig voneinander kombinierbar sind. Bevorzugte Substituenten im Benzol-, Pyridin-, Pyrazol-, Thiophen- oder (Alkylsulfonyl)alkylamino-Teil sind Alkyl, Alkoxy, Halogen, Nitro, Alkoxycarbonyl, Aminocarbonyl, Alkylaminocarbohyl, Dialkylaminocarbonyl, Alkyl, Alkylsulfonyl, Haloalkoxy, Haloalkyl, Alkylcarbonyl, Alkoxyalkyl, (Alkansulfonyl)alkylamino. Geeignete Sulfonylharnstoffe sind beispielsweise
   B1) Phenyl- und Benzylsulfonylharnstoffe und verwandte Verbindungen, z.B.
      1-(2-Chlorphenylsulfonyl)-3-(4-methoxy-6-methyl-1,3,5-triazin-2-yl)harnstoff (Chlorsulfuron),
      1-(2-Ethoxycarbonylphenylsulfonyl)-3-(4-chlor-6-methoxypyrimidin-2-yl)harnstoff (Chlorimuron-ethyl),
      1-(2-Methoxyphenylsulfonyl)-3-(4-methoxy-6-methyl-1,3,5-triazin-2-yl)harnstoff (Metsulfuron-methyl),
      1-(2-Chlorethoxy-phenylsulfonyl)-3-(4-methoxy-6-methyl-1,3,5-triazin-2-yl)harnstoff (Triasulfuron),
      1-(2-Methoxycarbonyl-phenylsulfonyl)-3-(4,6-dimethyl-pyrimidin-2-yl)harnstoff (Sulfometuron-methyl),
      1-(2-Methoxycarbonylphenylsulfonyl)-3-(4-methoxy-6-methyl-1,3,5-triazin-2-yl)-3-methylharnstoff (Tribenuron-methyl),
      1-(2-Methoxycarbonylbenzylsulfonyl)-3-(4,6-dimethoxy-pyrimidin-2-yl)harnstoff (Bensulfuron-methyl),
      1-(2-Methoxycarbonylphenylsulfonyl)-3-(4,6-bis-(difluormethoxy)pyrimidin-2-yl)harnstoff (Primisulfuron-methyl),
      3-(4-Ethyl-6-methoxy-1,3.5-triazin-2-yl)-1-(2,3-dihydro-1,1-dioxo-2-methylbenzo[b]thiophen-7-sulfonyl)-harnstoff (s. EP-A-79683),
      3-(4-Ethoxy-6-ethyl-1,3,5-triazin-2-yl)-1-(2,3-dihydro-1,1-dioxo-2-methylbenzo[b]thiophen-7-sulfonyl)-harnstoff (s. EP-A-79683),
      3-(4-Methoxy-6-methyl-1,3,5-triazin-2-yl)-1-(2-methoxycarbonyl-5-jodphenylsulfonyl)-harnstoff (s. WO 92/13845)
   B2) Thienylsulfonylharnstoffe, z.B. 1-(2-Methoxycarbonylthiophen-3-yl)-3-(4-methoxy-6-methyl-1,3,5-triazin-2-yl)harnstoff (Thifensulfuron-methyl),
   B3) Pyrazolylsulfonylharnstoffe, z.B.
      1-(4-Ethoxycarbonyl-1-methylpyrazol-5-yl-sulfonyl)-3-(4,6-dimethoxypyrimidin-2-yl)harnstoff (Pyrazosulfuron-methyl),
      3-Chlor-5-(4,6-dimethoxypyrimidin-2-ylcarbamoylsulfamoyl)-1-methyl-pyrazol-4-carbonsäuremethylester (s. EP 282613),
      5-(4,6-Dimethylpyrimidin-2-yl-carbamoylsulfamoyl)-1-(2-pyridyl)-pyrazol-4-carbonsäuremethylester (NC-330, s. Brighton Crop Prot. Conference - Weeds-1991, Vol. 1, 45 ff.),
   B4) Sulfondiamid-Derivate, z.B.
      3-(4,6-Dimethoxypyrimidin-2-yl)-1-(N-methyl-N-methylsulfonylaminosulfonyl)harnstoff
      (Amidosulfuron) und Strukturanaloge (s. EP-A-0131258 und Z. Pfl. Krankh. Pfl. Schutz 1990, Sonderheft XII, 489-497),
   B5) Pyridylsulfonylharnstoffe, z.B.
      1-(3-N,N-Dimethylaminocarbonylpyridin-2-yl-sulfonyl)-3-(4,6-dimethoxypyrimidin-2-yl)harnstoff (Nicosulfuron),
      1-(3-Ethylsulfonylpyridin-2-yl-sulfonyl)-3-(4,6-dimethoxy-pyrimidin-2-yl)harnstoff (DPX-E 9636, s. Brighton Crop Prot. Conf. - Weeds - 1989, S. 23 ff.), Pyridylsulfonylharnstoffe, wie sie in DE-A-4000503 und DE-A-4030577 beschrieben sind, vorzugsweise solche der Formel 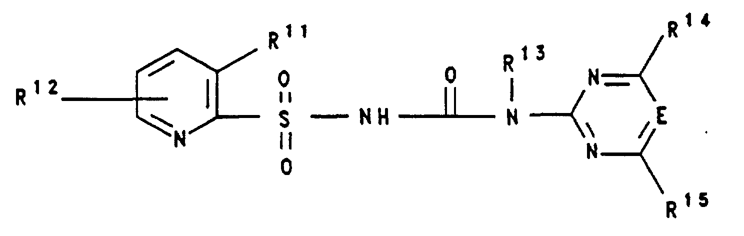 worin
         - E: CH oder N vorzugsweise CH,
         - R¹¹: lod oder NR¹⁶R¹⁷,
         - R¹²: H, Halogen, Cyano, C₁-C₃-Alkyl, C₁-C₃-Alkoxy, C₁-C₃-Haloalkyl, C₁-C₃-Haloalkoxy, C₁-C₃-Alkylthio, (C₁-C₃-Alkoxy)-C₁-C₃-alkyl,
         (C₁-C₃-Alkoxy)-carbonyl, Mono- oder Di-(C₁-C₃-alkyl)-amino, C₁-C₃-Alkyl-sulfinyl oder -sulfonyl, SO₂-NR^{a}R^{b} oder CO-NR^{a}R^{b}, insbesondere H,
         - R^{a},R^{b}: unabhängig voneinander H, C₁-C₃-Alkyl, C₁-C₃-Alkenyl, C₁-C₃-Alkinyl oder zusammen -(CH₂)₄-, -(CH₂)₅- oder (CH₂)₂-O-(CH₂)₂-,
         - R¹³: H oder CH₃,
         - R¹⁴: Halogen, C₁-C₂-Alkyl, C₁-C₂-Alkoxy, C₁-C₂-Haloalkyl, vorzugsweise CF₃, C₁-C₂-Haloalkoxy, vorzugsweise OCHF₂ oder OCH₂CF₃,
         - R¹⁵: C₁-C₂-Alkyl, C₁-C₂-Haloalkoxy, vorzugsweise OCHF₂, oder C₁-C₂-Alkoxy, und
         - R¹⁶: C₁-C₄-Alkyl und
         - R¹⁷: C₁-C₄-Alkylsulfonyl oder
         - R¹⁶ und R¹⁷: gemeinsam eine Kette der Formel -(CH₂)₃SO₂- oder -(CH₂)₄SO₂- bedeuten,
         z.B. 3-(4,6-Dimethoxypyrimidin-2-yl)-1-(3-N-methylsulfonyl-N-methylaminopyridin-2-yl)-sulfonylharnstoff, oder deren Salze,
   B6) Alkoxyphenoxysulfonylharnstoffe, wie sie in EP-A-0342569 beschrieben sind, vorzugsweise solche der Formel 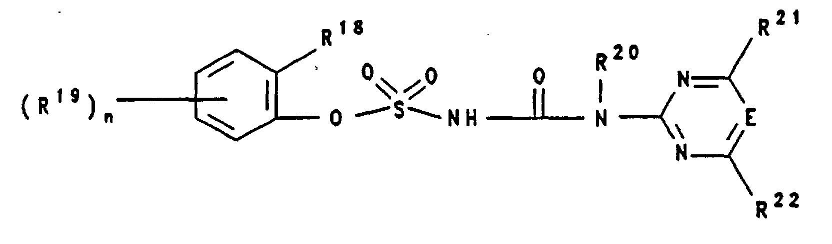
   worin
   - E: CH oder N, vorzugsweise CH,
   - R¹⁸: Ethoxy, Propoxy oder Isopropoxy,
   - R¹⁹: Wasserstoff, Halogen, NO₂, CF₃, CN, C₁-C₄-Alkyl, C₁-C₄-Alkoxy, C₁-C₄-Alkylthio oder (C₁-C₃-Alkoxy)-carbonyl, vorzugsweise in 6-Position am Phenylring,
   - n: 1, 2 oder 3, vorzugsweise 1,
   - R²⁰: Wasserstoff, C₁-C₄-Alkyl oder C₃-C₄-Alkenyl,
   - R²¹,R²²: unabhängig voneinander Halogen, C₁-C₂-Alkyl, C₁-C₂-Alkoxy, C₁-C₂-Haloalkyl, C₁-C₂-Haloalkoxy oder (C₁-C₂-Alkoxy)-C₁-C₂-alkyl, vorzugsweise OCH₃ oder CH₃, bedeuten, z.B. 3-(4,6-Dimethoxypyrimidin-2-yl)-1-(2-ethoxyphenoxy)-sulfonylharnstoff, oder deren Salze,
   und andere verwandte Sulfonylharnstoffderivate und Mischungen daraus,
C) Chloracetanilid-Herbizide wie
   N-Methoxymethyl-2,6-diethyl-chloracetanilid (Alachlor),
   N-(3'-Methoxyprop-2'-yl)-2-methyl-6-ethyl-chloracetanilid (Metolachlor),
   N-(3-Methyl-1,2,4-oxadiazol-5-yl-methyl)-chloressigsäure-2,6-dimethylanilid,
   N-(2,6-Dimethylphenyl)-N-(1-pyrazolylmethyl)-chloressigsäureamid
   (Metazachlor),
D) Thiocarbamate wie
   S-Ethyl-N,N-dipropylthiocarbamat (EPTC) oder
   S-Ethyl-N,N-diisobutylthiocarbamat (Butylate),
E) Cyclohexandion-Derivate wie
   3-(1-Allyloxyiminobutyl)-4-hydroxy-6,6-dimethyl-2- oxocyclohex-3-encarbonsäuremethylester (Alloxydim),
   2-(1-Ethoximinobutyl)-5-(2-ethylthiopropyl)-3-hydroxy-cyclohex-2-en-1-on (Sethoxydim),
   2-(1-Ethoximinobutyl)-5-(2-phenylthiopropyl)-3-hydroxy-cyclohex-2-en-1-on (Cloproxydim),
   2-(1-(3-Chlorallyloxy)iminobutyl)-5-[2-(ethylthio)propyl]-3-hydroxy-cyclohex-2-en-1-on,
   2-(1-(3-Chlorallyloxy)iminopropyl)-5-[2-(ethylthio)propyl]-3-hydroxy-cyclohex-2-en-1-on (Clethodim),
   2-(1-(Ethoxyimino)-butyl)-3-hydroxy-5-(thian-3-yl)-cyclohex-2-enon (Cycloxydim),
      oder
   2-(1-Ethoxyiminopropyl)-5-(2,4,6-trimethylphenyl)-3-hydroxy-cyclohex-2-en-1-on (Tralkoxydim),
F) 2-(4-Alkyl-5-oxo-2-imidazolin-2-yl)-benzoesäurederivate oder 2-(4-Alkyl-5-oxo-2-imidazolin-2yl)-heteroarylcarbonsäurederivate wie z.B.
   2-(4-Isopropyl-4-methyl-5-oxo-2-imidazolin-2-yl)-5-methylbenzoesäuremethylester und
   2-(4-lsopropyl-4-methyl-5-oxo-2-imidazolin-2-yl)-4-methylbenzoesäure (Imazamethabenz),
   5-Ethyl-2-(4-isopropyl-4-methyl-5-oxo-2-imidazolin-2-yl)-pyridin-3-carbonsäure (Imazethapyr),
   2-(4-Isopropyl-4-methyl-5-oxo-2-imidazolin-2-yl)-chinolin-3-carbonsäure (Imazaquin),
   2-(4-Isopropyl-4-methyl-5-oxo-2-imidazolin-2-yl)-pyridin-3-carbonsäure (Imazapyr),
   5-Methyl-2-(4-Isopropyl-4-methyl-5-oxo-2-imidazolin-2-yl)-pyridin-3-carbonsäure (Imazethamethapyr),
G) Triazolopyrimidinsulfonamidderivate, z. B.
   N-(2,6-Difluorphenyl)-7-methyl-1,2,4-triazolo-(1,5-c)-pyrimidin-2-sulfonamid (Flumetsulam),
   N-(2,6-Dichlor-3-methylphenyl)-5,7-dimethoxy-1,2,4-triazolo-(1,5-c)-pyrimidin-2-sulfonamid,
   N-(2,6-Difluorphenyl)-7-fluor-5-methoxy-1,2,4-triazolo-(1,5-c)-pyrimidin-2-sulfonamid
   N-(2,6-Dichlor-3-methylphenyl)-7-chlor-5-methoxy-1,2,4-triazolo-(1,5- c)-pyrimidin-2-sulfonamid,
   N-(2-Chlor-6-methoxycarbonyl)-5,7-dimethyl-1,2,4-triazolo-(1,5-c)-pyrimidin-2-sulfonamid
   (siehe z. B. EP-A-343 752, US- 4 988 812),
   H) Benzoylcyclohexandionderivate, z. B.
   2-(2-Chlor-4-methylsulfonylbenzoyl)-cyclohexan-1,3-dion (SC-0051, s. EP-A-137963),
   2-(2-Nitrobenzoyl)-4,4-dimethyl-cyclohexan-1,3-dion (s. EP-A-274634),
   2-(2-Nitro-3-methylsulfonylbenzoyl)-4,4-dimethyl-cyclohexan-1,3-dion (s. WO-91/13548),
J) Pyrimidinyloxy-pyrimidincarbonsäure- bzw. Pyrimidinyloxy-benzoesäure-Derivate, z.B.
   3-(4,6-Dimethoxypyrimidin-2-yl)-oxy-pyridin-2-carbonsäurebenzylester (EP-A-249 707),
   3-(4,6-Dimethoxypyrimidin-2-yl)-oxy-pyridin-2-carbonsäuremethylester (EP-A-249 707),
   2,6-Bis[(4,6-dimethoxypyrimidin-2-yl)-oxy]-benzoesäure (EP-A-321 846),
   2,6-Bis[(4,6-dimethoxypyrimidin-2-yl)-oxy]-benzoesäure-(1-ethoxycarbonyloxyethyl)-ester (EP-A-472 113) und
K) S-(N-Aryl-N-alkyl-carbamoylmethyl)-dithiophosphorsäureester wie S-[N-(4-Chlorphenyl)-N-isopropyl-carbamoylmethyl]-O,O-dimethyl-dithiophosphat (Anilofos).

Die obengenannten Herbizide der Gruppen A bis K sind dem Fachmann bekannt und in der Regel in "The Pesticide Manual", British Crop Protection Council, 9. Auflage 1991 oder 8. Auflage 1987 oder in "Agricultural Chemicals Book II, Herbicides", by W.T. Thompson, Thompson Publications, Fresno CA, USA 1990 oder in "Farm Chemicals Handbook '90", Meister Publishing Company, Willoughby OH, USA 1990 beschrieben. Imazethamethapyr ist aus Weed Techn. 1991, Vol. 5, 430-438 bekannt.

Die herbiziden Wirkstoffe und die erwähnten Safener können zusammen (als fertige Formulierung oder im Tank-mix-Verfahren) oder in beliebiger Reihenfolge nacheinander ausgebracht werden. Das Gewichtsverhältnis Safener:Herbizid kann innerhalb weiter Grenzen variieren und ist vorzugsweise im Bereich von 1:10 bis 10:1, insbesondere von 1:10 bis 5:1. Die jeweils optimalen Mengen an Herbizid und Safener sind vom Typ des verwendeten Herbizids oder vom verwendeten Safener sowie von der Art des zu behandelnden Pflanzenbestandes abhängig und lassen sich von Fall zu Fall durch entsprechende Vorversuche ermitteln. Entsprechende Mengenverhältnisse sind bei Anwendung von Safener und anderen Pflanzenschutzmittelwirkstoffen, wie Insektiziden oder Insektid-Herbizid-Kombinationen sinnvoll.

Haupteinsatzgebiete für die Anwendung der Safener sind vor allem Getreidekulturen (Weizen, Roggen, Gerste, Hafer), Reis, Mais, Sorghum, aber auch Baumwolle und Sojabohne, vorzugsweise Getreide, Reis und Mais.

Ein besonderer Vorteil der erfindungsgemäßen Safener der Formel (I) ist bei deren Kombination mit Herbiziden aus der Gruppe der Sulfonylharnstoffe und/oder Imidazolinone sowie mit Herbiziden vom Typ der Phenoxyphenoxyund Heteroaryloxy-phenoxy-alkancarbonsäurederivate festzustellen.

Einige Herbizide dieser Strukturklassen können speziell in Getreidekulturen und/oder Mais sowie Reis nicht oder nicht genügend selektiv eingesetzt werden. Durch die Kombination mit den erfindungsgemäßen Safenern sind auch bei diesen Herbiziden in Getreide, Mais oder Reis hervorragende Selektivitäten zu erreichen.

Die Safener der Formel (I) können je nach ihren Eigenschaften zur Vorbehandlung des Saatgutes der Kulturpflanze (Beizung der Samen) verwendet werden oder vor der Saat in die Saatfurchen eingebracht oder zusammen mit dem Herbizid vor oder nach dem Auflaufen der Pflanzen angewendet werden. Vorauflaufbehandlung schließt sowohl die Behandlung der Anbaufläche vor der Aussaat als auch die Behandlung der angesäten, aber noch nicht bewachsenen Anbauflächen ein. Bevorzugt ist die gemeinsame Anwendung mit dem Herbizid, insbesondere in Nachauflaufverfahren. Hierzu können Tankmischungen oder Fertigformulierungen eingesetzt werden.

Die benötigten Aufwandmengen der Safener können je nach Indikation und verwendetem Herbizid innerhalb weiter Grenzen schwanken und liegen in der Regel im Bereich von 0,001 bis 5 kg, vorzugsweise 0,005 bis 0,5 kg Wirkstoff je Hektar.

Gegenstand der vorliegenden Erfindung ist deshalb auch ein Verfahren zum Schutz von Kulturpflanzen vor phytotoxischen Nebenwirkungen von Pestiziden, vorzugsweise von Herbiziden, das dadurch gekennzeichnet ist, daß eine wirksame Menge einer Verbindung der Formel (I) vor, nach oder gleichzeitig mit dem Pestizid bzw. Herbizid auf die Pflanzen, Pflanzensamen oder die Anbaufläche appliziert wird.

Gegenstand der Erfindung sind auch pflanzenschützende Mittel, die einen Wirkstoff der Formel (I) und übliche Formulierungshilfsmittel enthalten, sowie pestizide, vorzugsweise herbizide Mittel, die einen Wirkstoff der Formel (I) und ein Pestizid bzw. Herbizid sowie im Bereich des Pflanzenschutzes übliche Formulierungshilfsmittel enthalten.

Die Verbindungen der Formel (I) und deren Kombinationen mit einem oder mehreren der genannten Herbizide können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), emulgierbare Konzentrate (EC), wasserlösliche Pulver (SP), wasserlösliche Konzentrate (SL), konzentrierte Emulsionen (EW) wie Öl-in-Wasser und Wasser-in- Öl-Emulsionen, versprühbare Lösungen oder Emulsionen, Kapselsuspensionen (CS), Dispersionen auf Öl- oder Wasserbasis (SC), Suspoemulsionen, Suspensionskonzentrate, Stäubemittel (DP), ölmischbare Lösungen (OL), Beizmittel, Granulate (GR) in Form von Mikro-, Sprüh-, Aufzugs- und Adsorptionsgranulaten, Granulate für die Boden- bzw. Streuapplikation, wasserlösliche Granulate (SG), wasserdispergierbare Granulate (WG), ULV-Formulierungen, Mikrokapseln und Wachse.

Diese einzelnen Formulierungstypen sind im Prinzip bekannt und werden beispielsweise beschrieben in: Winnacker-Küchler, "Chemische Technologie" Band 7, C. Hauser Verlag München, 4. Aufl. 1986; Wade van Valkenburg, "Pesticide Formulations", Marcel Dekker N.Y., 1973; K. Martens, "Spray Drying Handbook", 3rd Ed. 1979, G. Goodwin Ltd. London.

Die notwendigen Formulierungshilfsmittel wie Inertmaterialien. Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J.; H.v.Olphen, "Introduction to Clay Colloid Chemistry", 2nd Ed., J. Wiley & Sons, N.Y.; Marsden, "Solvents Guide", 2nd Ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler "Chemische Technolgie", Band 7, C. Hauser Verlag München, 4. Aufl. 1986.

Auf der Basis dieser Formulierungen lassen sich auch Kombinationen mit anderen pestizid wirksamen Stoffen, Düngemitteln und/oder Wachstumsregulatoren herstellen, z.B. in Form einer Fertigformulierung oder als Tankmix.

Spritzpulver sind in Wasser gleichmäßig dispergierbare Präparate, die neben dem Wirkstoff außer einem Verdünnungs- oder Inertstoff noch Netzmittel, z.B. polyoxethylierte Alkylphenole, polyoxethylierte Fettalkohole und Fettamine, Fettalkoholpolyglykolethersulfate, Alkansulfonate oder Alkylarylsulfonate und Dispergiermittel, z.B. ligninsulfonsaures Natrium, 2,2'-dinaphthylmethan-6,6'-disulfonsaures Natrium, dibutylnaphthalinsulfonsaures Natrium oder auch oleoylmethyltaurinsaures Natrium enthalten.

Emulgierbare Konzentrate werden durch Auflösen des Wirkstoffes in einem organischen Lösungsmittel, z.B. Butanol, Cyclohexanon, Dimethylformamid, Xylol oder auch höhersiedenden Aromaten oder Kohlenwasserstoffen unter Zusatz von einem oder mehreren Emulgatoren hergestellt. Als Emulgatoren können beispielsweise verwendet werden: Alkylarylsulfonsaure Calcium-Salze wie Ca-Dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte (z.B. Blockpolymere), Alkylpolyether, Sorbitanfettsäureester, Polyoxyethylensorbitanfettsäureester oder Polyoxethylensorbitester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Die agrochemischen Zubereitungen enthalten in der Regel 0,1 bis 99 Gewichtsprozent, insbesondere 0,1 bis 95 Gew.-%, Wirkstoffe der Formel (I) (Antidot) oder des Antidot/Herbizid-Wirkstoffgemischs und 1 bis 99,9 Gew.-%, insbesondere 5 bis 99,8 Gew.-%, eines festen oder flüssigen Zusatzstoffes und 0 bis 25 Gew.-%, insbesondere 0,1 bis 25 Gew.-%, eines Tensides.

In Spritzpulvern beträgt die Wirkstoffkonzentration z.B. etwa 10 bis 90 Gew.%, der Rest zu 100 Gew.-% besteht aus üblichen Formulierungsbestandteilen. Bei emulgierbaren Konzentraten beträgt die Wirkstoffkonzentration etwa 1 bis 80 Gew.-% Wirkstoffe. Staubförmige Formulierungen enthalten etwa 1 bis 20 Gew.-% an Wirkstoffen, versprühbare Lösungen etwa 0,2 bis 20 Gew.-% Wirkstoffe. Bei Granulaten wie wasserdispergierbaren Granulaten hängt der Wirkstoffgehalt zum Teil davon ab, ob die wirksame Verbindung flüssig oder fest vorliegt. In der Regel liegt der Gehalt bei den in Wasser dispergierbaren Granulaten zwischen 10 und 90 Gew.-%.

Daneben enthalten die genannten Wirkstofformulierungen gegebenenfalls die jeweils üblichen Haft-, Netz-, Dispergier- , Emulgier-, Penetrations-, Lösungsmittel, Füll- oder Trägerstoffe.

Zur Anwendung werden die in handelsüblicher Form vorliegenden Formulierungen gegebenenfalls in üblicher Weise verdünnt, z.B. bei Spritzpulvern, emulgierbaren Konzentraten, Dispersionen und wasserdispergierbaren Granulaten mittels Wasser. Staubförmige Zubereitungen, Granulate sowie versprühbare Lösungen werden vor der Anwendung üblicherweise nicht mehr mit weiteren inerten Stoffen verdünnt. Mit den äußeren Bedingungen wie Temperatur, Feuchtigkeit, der Art des verwendeten Pestizids bzw. Herbizids u.a. variiert die erforderliche Aufwandmenge der "Antidots".

Folgende Beispiele dienen zur Erläuterung der Erfindung:
A. Formulierungsbeispiele
   a) Ein Stäubmittel wird erhalten, indem man 10 Gew.-Teile einer Verbindung der Formel (I) oder eines Wirkstoffgemischs aus einem Herbizid und eine Verbindung der Formel (I) und 90 Gew.-Teile Talkum als Inertstoff mischt und in einer Schlagmühle zerkleinert.
   b) Ein in Wasser leicht dispergierbares, benetzbares Pulver wird erhalten, indem man 25 Gewichtsteile einer Verbindung der Formel (I) oder eines Wirkstoffgemischs aus einem Herbizid und einem Safener der Formel (I), 64 Gewichtsteile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.
   c) Ein in Wasser leicht dispergierbares Dispersionskonzentrat wird erhalten, indem man 20 Gewichtsteile einer Verbindung der Formel (I) oder eines Wirkstoffgemischs aus einem Herbizid und einem Safener der Formel (I), 6 Gew.-Teilen Alkylphenolpolyglykolether (^{R}Triton X 207), 3 Gew.-Teilen Isotridecanolpolyglykolether (8 EO) und 71 Gew.- Teilen paraffinischem Mineralöl (Siedebereich z.B. ca. 255 bis über 277°C) mischt und in einer Reibkugelmühle auf eine Feinheit von unter 5 Mikron vermahlt.
   d) Ein emulgierbares Konzentrat wird erhalten aus 15 Gew.-Teilen einer Verbindung der Formel (I) oder eines Wirkstoffgemischs aus einem Herbizid und einem Safener der Formel (I), 75 Gew.-Teilen Cyclohexanon als Lösemittel und 10 Gew.-Teilen oxethyliertem Nonylphenol als Emulgator.
   e) Ein in Wasser dispergierbares Granulat wird erhalten, indem man
      - 75 Gew.-Teile: einer Verbindung der Formel (I) oder eines Wirkstoffgemischs aus einem Herbizid und einem Safener der Formel (I),
      - 10 ": ligninsulfonsaures Calcium,
      - 5 ": Natriumlaurylsulfat,
      - 3 ": Polyvinylalkohol und
      - 7 ": Kaolin
      mischt, auf einer Stiftmühle mahlt und das Pulver in einem Wirbelbett durch Aufsprühen von Wasser als Granulierflüssigkeit granuliert.
   f) Ein in Wasser dispergierbares Granulat wird auch erhalten, indem man
      - 25 Gew.-Teile: einer Verbindung der Formel (I) oder eines Wirkstoffgemischs aus einem Herbizid und einem Safener der Formel (I),
      - 5 ": 2,2'-dinaphthylmethan-6,6'-disulfonsaures Natrium,
      - 2 ": oleoylmethyltaurinsaures Natrium,
      - 1 ": Polyvinylalkohol,
      - 17 ": Calciumcarbonat und
      - 50 ": Wasser
      auf einer Kolloidmühle homogensiert und vorzerkleinert, anschließend auf einer Perlmühle mahlt und die so erhaltene Suspension in einem Sprühturm mittels einer Einstoffdüse zerstäubt und trocknet.
B. Herstellungsbeispiele
   1. 5,5-Diphenyl-2-isoxazolin-3-carbonsäureethylester
      13,52 g (0,075 mol) 1,1-Diphenylethen und 5,06 g (0,05 mol) Triethylamin werden bei O°C in 200 ml Ether gelöst, anschließend werden in ca. zwei Stunden 7,58 g (0,05 mol) 2-Chlor-2-hydroximino-essigsäureethylester, gelöst in 100 ml Ether, zugetropft. Nach einstündigem Nachrühren bei Raumtemperatur werden 100 ml H₂O zugegeben und anschließend wird das Gemisch mit Ether extrahiert. Nach Trocknen über MgSO₄ wird der Ether abdestilliert und der Rückstand über eine Kieselgelsäule (Laufmittel: n-Heptan:Essigester = 8:2) gereinigt. So werden 12,7 g (86 % d. Th.) Produkt mit dem Schmelzpunkt 78 bis 81°C erhalten.

Die Verbindungen der folgenden Tabelle 1 werden auf analogem Weg zu Beispiel 1 bzw. den weiter oben beschriebenen Methoden erhalten.

### Abkürzungen in Tabelle 1:

- Me =: Methyl
- Bu =: Butyl
i-, s-, t-, c-Alkyl = iso-, sekundär-, tertiär- bzw. cyclo-Alkyl Schmp. = Schmelzpunkt (in °C)

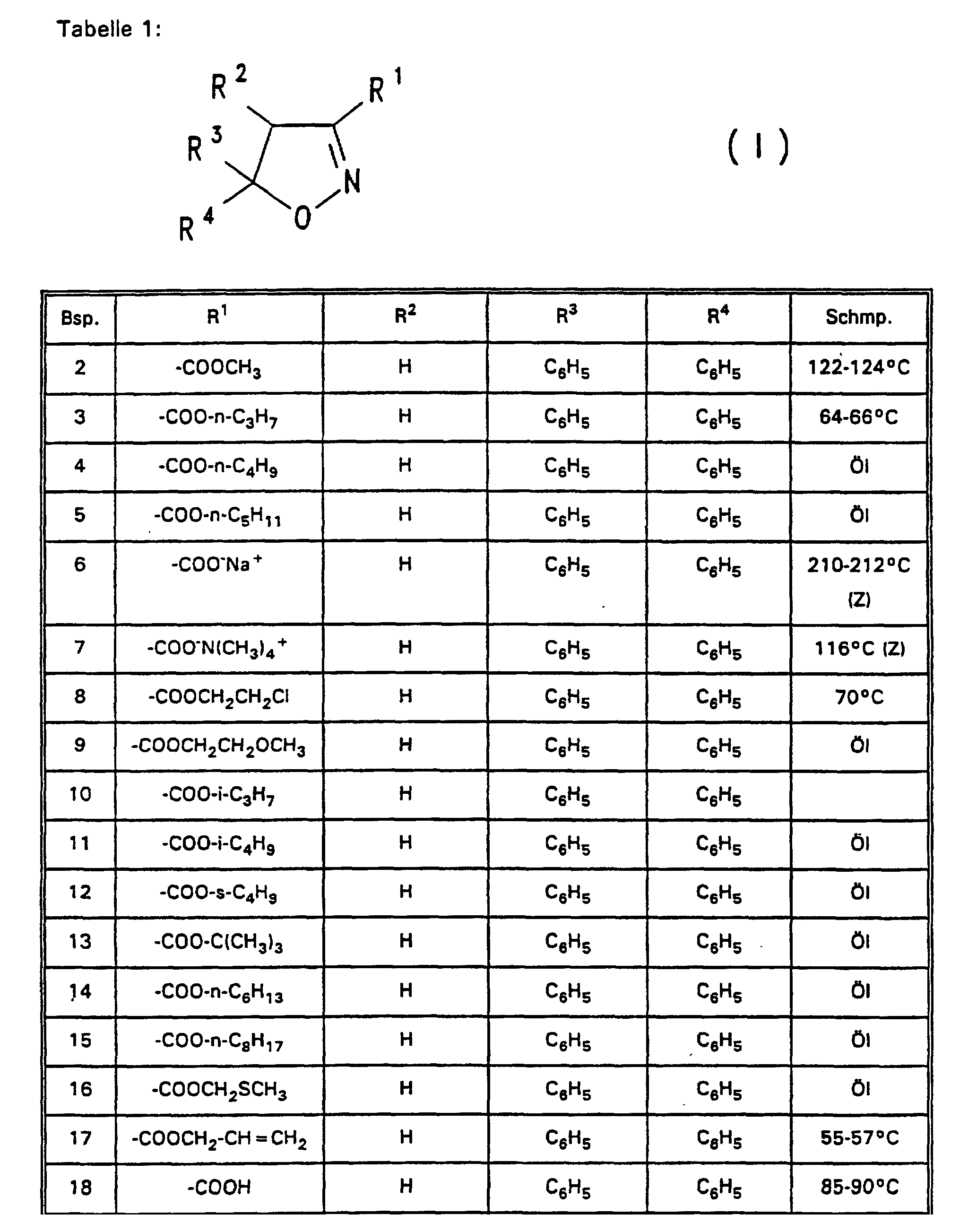

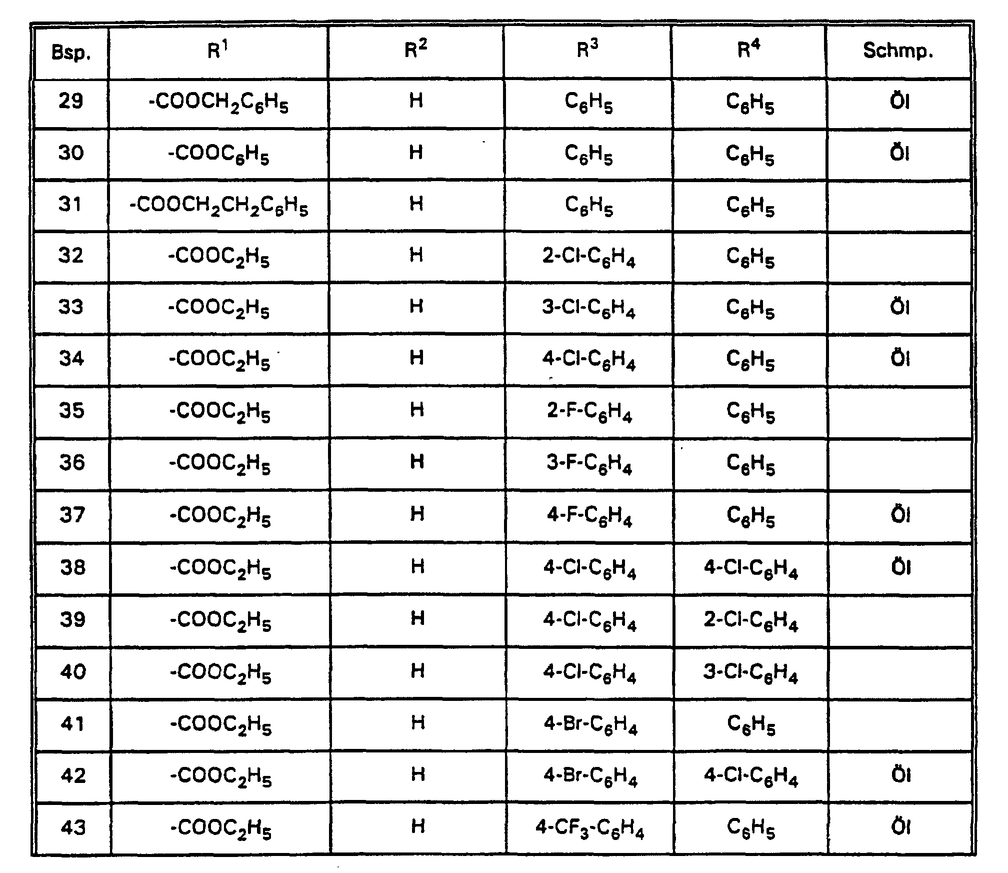

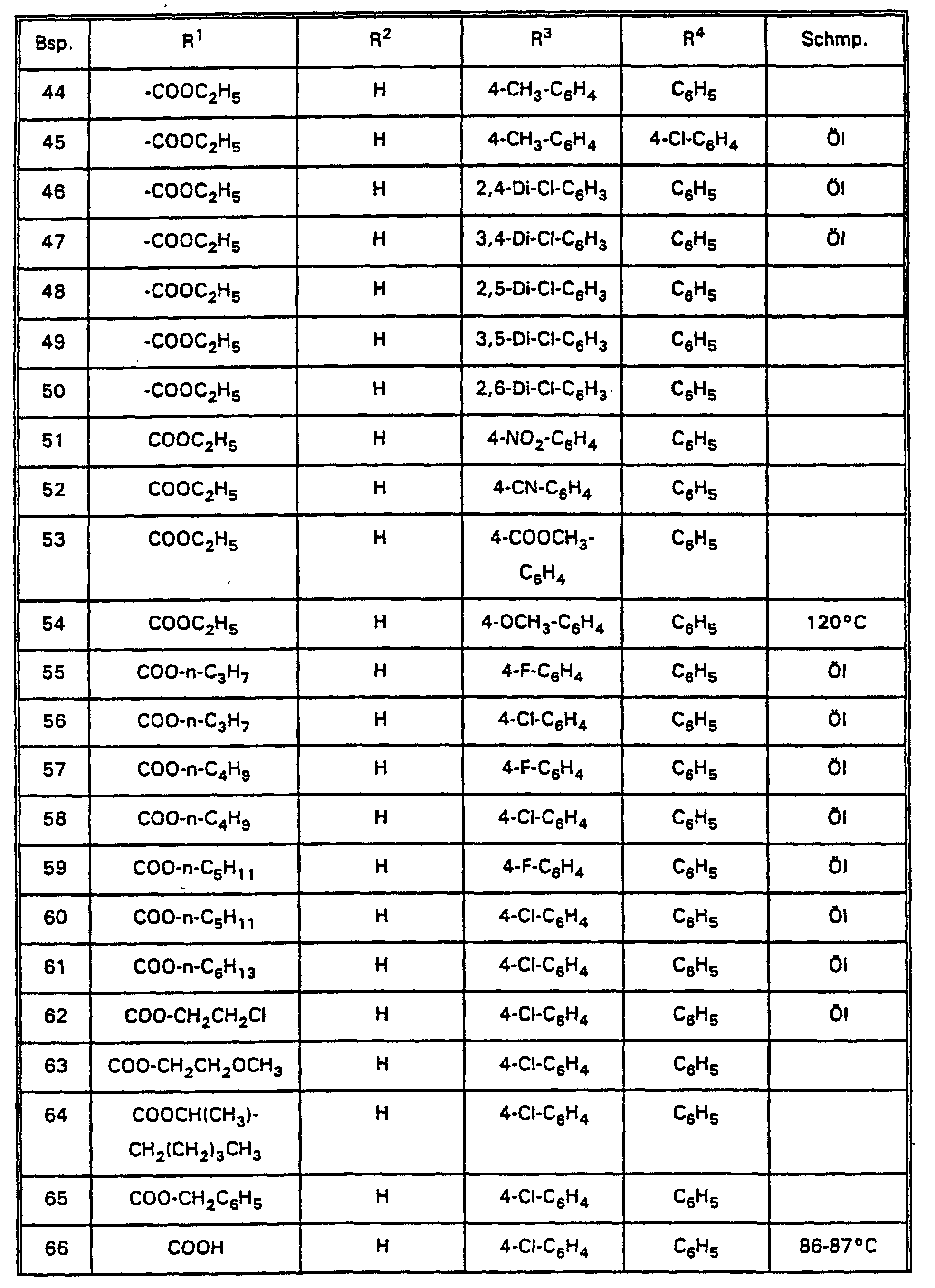

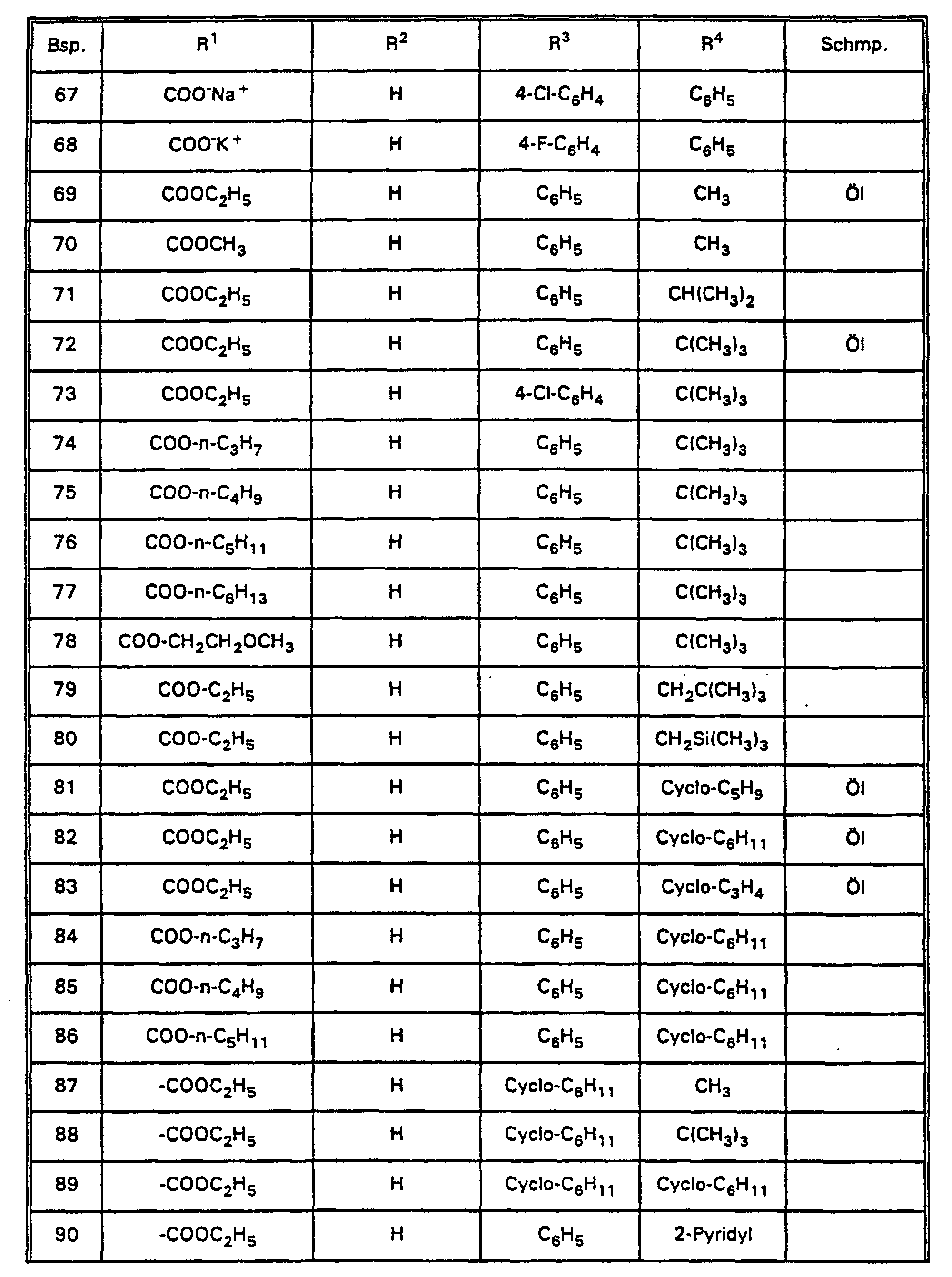

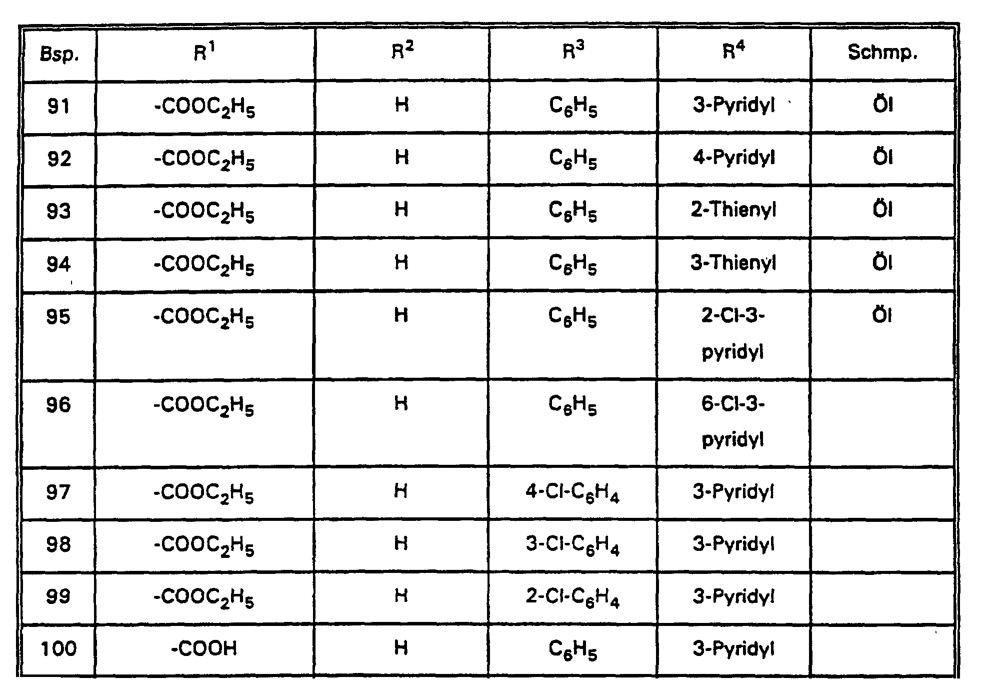

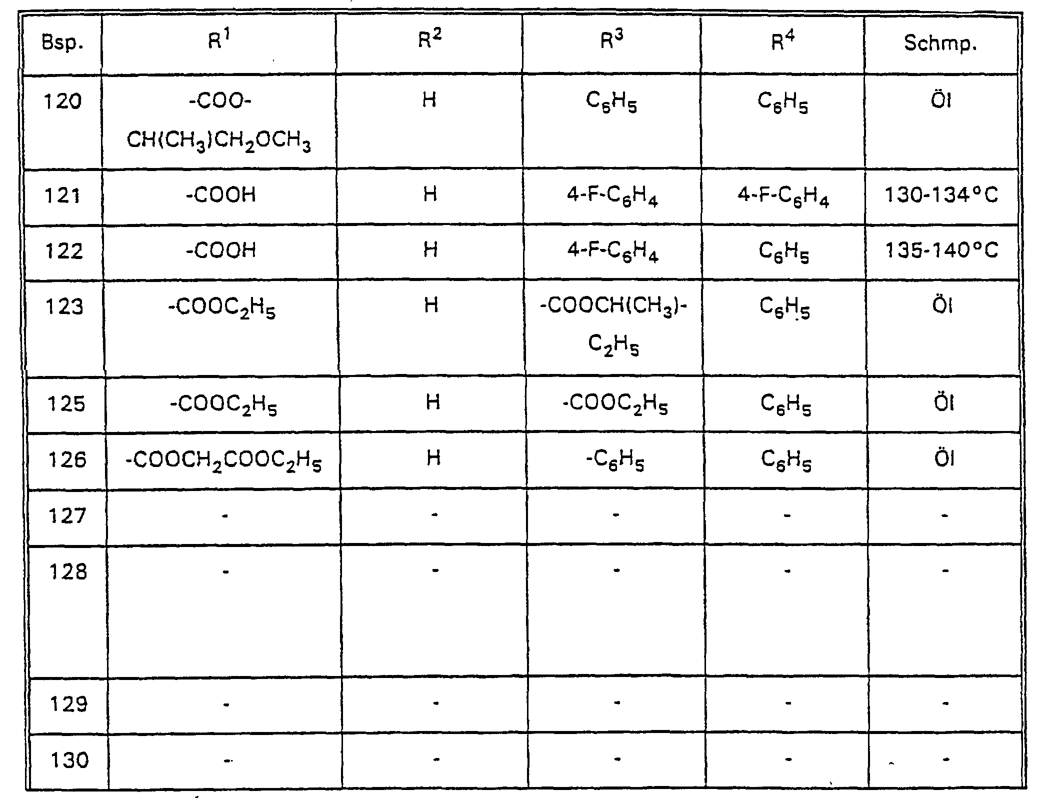

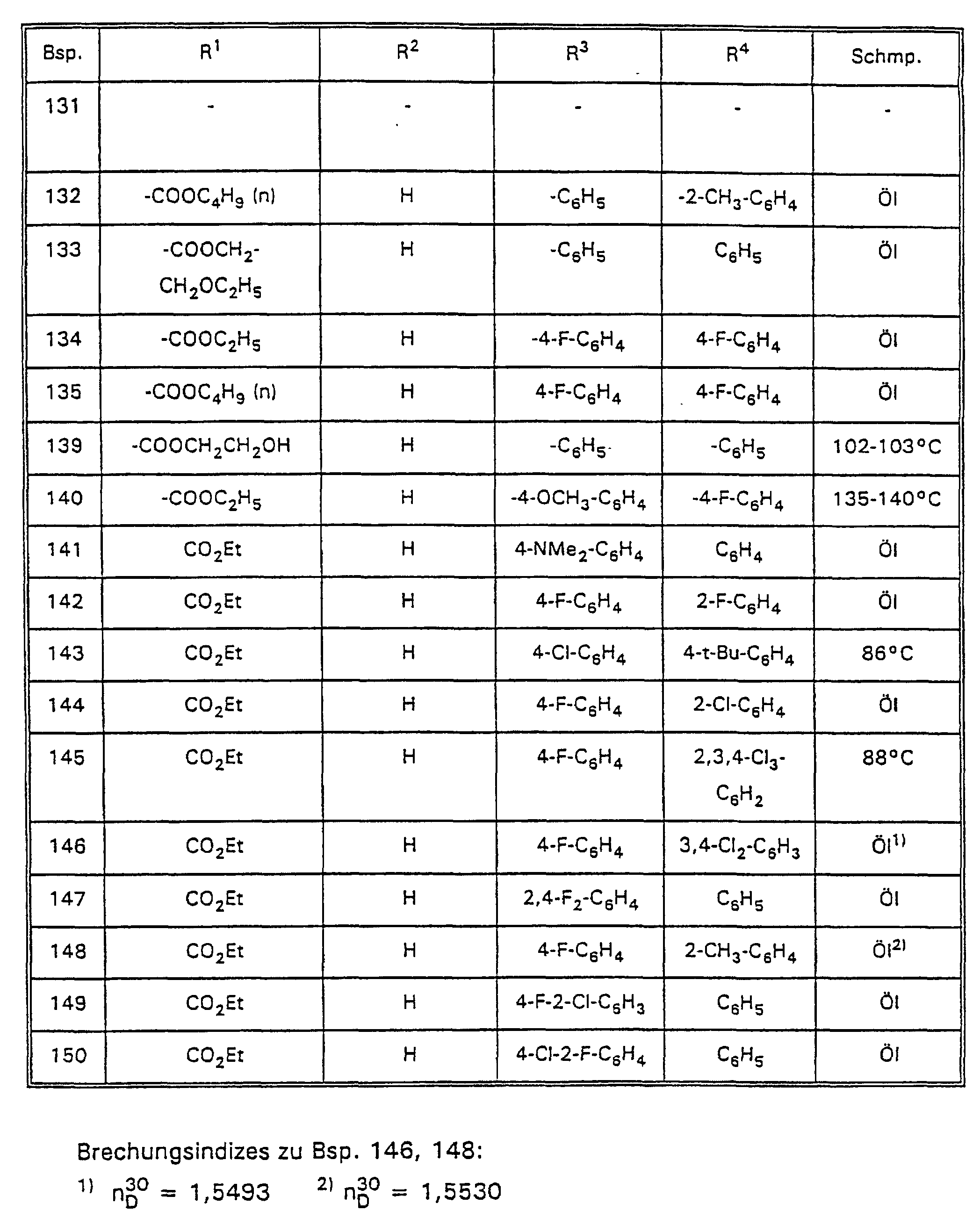

### C. Biologische Beispiele

Samen von Weizen, Gerste oder Reis werden in sandiger Lehmerde in Plastiktöpfen ausgelegt, im Gewächshaus bis zum 3- bis 4- Blattstadium herangezogen und dann nacheinander mit den erfindungsgemäßen Verbindungen und den Herbiziden im Nachlaufverfahren behandelt. Die Herbizide und die Verbindungen der Formel (I) werden dabei in Form wäßriger Suspensionen bzw. Emulsionen mit einer Wasseraufwandmenge von umgerechnet 300 I/ha ausgebracht. 3-4 Wochen nach der Behandlung werden die Pflanzen visuell auf jede Art von Schädigung durch die ausgebrachten Herbizide bonitiert, wobei insbesondere das Ausmaß der anhaltenden Wachstumshemmung berücksichtigt wird. Die Bewertung erfolgt in Prozentwerten im Vergleich zu unbehandelten Kontrollen.
Einige Versuchsergebnisse sind in Tabellen 2, 3 und 4 zusammengestellt.

**Tabelle 2:**

| Safenerwirkung in Gerste | | |
|---|---|---|
| Produkt Herbizid/Safener | Dosis (kg a.i./ha) | herbizide Wirkung in % HOVU |
| | 0,2 | 80 |
| H₁ + Nr. 1 | 0,2 + 1,25 | 60 |
| H₁ + Nr. 2 | 0,2 + 1,25 | 60 |
| H₁ + Nr. 6 | 0,2 + 1,25 | 20 |
| H₁ + Nr. 17 | 0,2 + 1,25 | 20 |
| H₁ + Nr. 4 | 0,2 + 1,25 | 20 |
| H₁ + Nr. 3 | 0,2 + 1,25 | 30 |
| H₁ + Nr. 7 | 0,2 + 1,25 | 37 |
| H₁ = Fenoxaprop-P-ethyl | | |
| HOVU = Hordeum vulgare (Gerste) | | |
| Nr. ... = Safener von Beispiel Nr. ... aus Abschnitt B (Chemische Beispiele) | | |

**Tabelle 3:**

| Safenerwirkung in Reis | | |
|---|---|---|
| Produkt Herbizid/Safener | Dosis (kg a.i./ha) | herbizide Wirkung in % ORSA |
| H₁ | 0,3 | 75 |
| H₁ + Nr. 1 | 0.3 + 1,25 | 60 |
| H₁ + Nr. 2 | 0,3 + 1,25 | 70 |
| H₁ + Nr. 6 | 0,3 + 1,25 | 70 |
| H₁ + Nr. 17 | 0,3 + 1,25 | 70 |
| H₁ + Nr. 4 | 0,3 + 1,25 | 65 |
| H₁ + Nr. 3 | 0,3 + 1,25 | 20 |
| H₁ + Nr. 7 | 0,3 + 1,25 | 70 |
| H₂ = Fenoxaprop-P-ethyl | | |
| ORSA = Oryza sativa (Reis) | | |

**Tabelle 4:**

| Safenerwirkung in Mais | | |
|---|---|---|
| Produkt Herbizid/Safener | Dosis (kg a.i./ha) | herbizide Wirkung in % ZEMV |
| H₂ | 0,075 | 70 |
| H₂ + Nr. 1 | 0,075 + 1,25 | 20 |
| H₂ + Nr. 2 | 0,075 + 1,25 | 30 |
| H₂ + Nr. 6 | 0,075 + 1,25 | 50 |
| H₂ + Nr. 17 | 0,075 + 1,25 | 70 |
| H₂ + Nr. 4 | 0,075 + 1,25 | 30 |
| H₂ + Nr. 3 | 0,075 + 1,25 | 40 |
| H₂ + Nr. 7 | 0,075 + 1,25 | 30 |
| H₂ = 3-(4,6-Dimethoxypyrimidin-2-yl)-1-(3-N-methylsulfonyl-N-methylaminopyridin-2-yl)-sulfonylharnstoff | | |
| ZEMV = Zea mays (Mais) | | |

### Beispiel 2

Maispflanzen werden im Gewächshaus in Plastiktöpfen bis zum 4-Blattstadium bzw. bis zum 6-Blattstadium herangezogen und mit einer Tankmischung, bestehend aus einem Herbizid und erfindungsgemäßen Verbindungen der Formel (I), behandelt. Die Präparate werden bei einer Wasseraufwandmenge von 300 l/ha auf die wachsenden Pflanzen gesprüht. 4 Wochen nach Behandlung werden die Pflanzen auf vorhandene Phytotoxizität bonitiert und das Ausmaß der Schädigung im Vergleich zur unbehandelten Kontrolle bestimmt.

Die Versuchsergebnisse, dargestellt in Tabellen 5 und 6, zeigen, daß die erfindungsgemäßen Verbindungen Pflanzenschädigungen sehr wirksam verhindern können.

**Tabelle 5:**

| Wirkung der erfindungsgemäßen Verbindungen an Maispflanzen | | | |
|---|---|---|---|
| Stoffe | Dosis | herbizide Wirkung bei Mais (in %) | |
| Herbizid/Safener | kg AS/ha | 4-Blattstadium | 6-Blattstadium |
| H₂ | 0,200 | 77 | 83 |
| | 0,100 | 70 | 73 |
| | 0,050 | 63 | 60 |
| | 0,025 | 33 | 40 |
| H₂ + Nr. 1 | 0,200 0,200 | 5 | 10 |
| | 0,100 0,100 | 0 | 0 |
| | 0,050 0,050 | 0 | 0 |
| | 0.025 0,025 | 0 | 0 |
| H₂ + Nr. 3 | 0,200 0,200 | 40 | 0 |
| | 0,100 0,100 | 20 | 0 |
| | 0,050 0,050 | 0 | 0 |
| | 0,025 0,025 | 0 | 0 |
| H₂ + Nr. 17 | 0,200 0,200 | 20 | 10 |
| | 0,100 0,100 | 10 | 0 |
| | 0,050 0,050 | 0 | 0 |
| | 0,025 0,025 | 0 | 0 |
| H₂ + Nr. 6 | 0,200 0,200 | 27 | 30 |
| | 0,100 0,100 | 7 | 20 |
| | 0,050 0,050 | 0 | 10 |
| | 0,025 0,025 | 0 | 0 |
| H₂ + Nr. 7 | 0,200 0,200 | 20 | 33 |
| | 0,100 0,100 | 0 | 20 |
| | 0,050 0,050 | 0 | 0 |
| | 0,025 0,025 | 0 | 0 |
| H₂ + Nr. 4 | 0,200 0,200 | 20 | 0 |
| | 0,100 0,100 | 0 | 0 |
| | 0,050 0,050 | 0 | 0 |
| | 0,025 0,025 | 0 | 0 |
| H₂ = 3-(4,6-Dimethoxypyrimidin-2-yl)-1-(3-N-methylsulfonyl-N-methylaminopyridin-2-yl)-sulfonylharnstoff | | | |

**Tabelle 6:**

| Wirkung der erfindungsgemäßen Verbindungen an Maispflanzen | | | |
|---|---|---|---|
| Stoffe | Dosis | herbizide Wirkung bei Mais (in %) | |
| Herbizid/Safener | kg AS/ha | 4-Blattstadium | 6-Blattstadium |
| H₃ | 0,200 | 90 | 88 |
| | 0,100 | 80 | 80 |
| | 0,050 | 75 | 80 |
| | 0,025 | 60 | 65 |
| H₃ + Nr. 3 | 0,200 0,200 | 5 | 10 |
| | 0,100 0,100 | 0 | 0 |
| | 0,050 0,050 | 0 | 0 |
| | 0,025 0,025 | 0 | 0 |
| H₃ + Nr. 4 | 0,200 0,200 | 10 | 15 |
| | 0,100 0,100 | 0 | 10 |
| | 0,050 0,050 | 0 | 0 |
| | 0,025 0,025 | 0 | 0 |
| H₃ + Nr. 7 | 0,200 0,200 | 20 | 25 |
| | 0,100 0,100 | 0 | 10 |
| | 0,050 0,050 | 0 | 0 |
| | 0,025 0,025 | 0 | 0 |
| H₃ = 3-(4-Methoxy-6-methyl-1,3,5-triazin-2-yl)-1-(2-methoxycarbonyl-5-jod-phenylsulfonyl)-harnstoff | | | |

## Patentansprüche

1. Pflanzenschutzmittel, **dadurch gekennzeichnet, daß** es als kulturpflanzenschützende Komponente eine Verbindung der Formel (I) oder deren Salz, 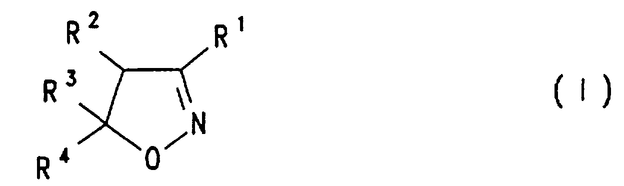 worin
R¹ einen Acylrest aus der Gruppe der Reste der Formel 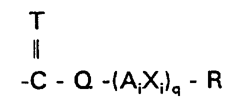 und 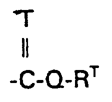 worin R, R^{T}, T, Q, Aᵢ, Xᵢ und q wie weiter unten definiert sind,
R² Wasserstoff,
R³ und R⁴ unabhängig voneinander C₁-C₁₈-Alkyl, C₃-C₁₂-Cycloalkyl, C₂-C₈-Alkenyl oder C₂-C₈-Alkinyl oder einen Rest der Formel 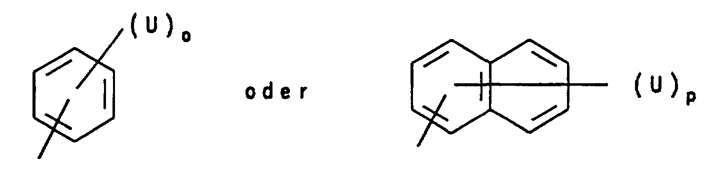 worin
(U) für gleiche oder verschiedene Reste stehen, welche unabhängig voneinander Wasserstoff, Halogen, Cyano, Nitro, Amino oder C₁-C₈-Haloalkyl, C₁-C₈-Haloalkoxy, C₁-C₈-Alkyl, C₁-C₈-Alkoxy, Mono-(C₁-C₄-alkyl)-amino, Di-(C₁-C₄-alkyl)-amino, C₁-C₈-Alkylthio oder C₁-C₈-Alkylsulfonyl, wobei jeder der letztgenannten 8 Reste unsubstituiert oder durch einen oder mehrere gleiche oder verschiedene Substituenten aus der Gruppe enthaltend Halogen, C₁-C₈-Haloalkoxy, Nitro, Cyano, Hydroxy, C₁-C₈-Alkoxy und eine C₁-C₈-Alkoxygruppe, worin eine oder mehrere CH₂-Gruppen durch Sauerstoff ersetzt sind, und C₁-C₈-Alkylthio, C₁-C₆-Alkylsulfinyl, C₁-C₆-Alkylsulfonyl, C₂-C₈-Alkenylthio, C₂-C₈-Alkinylthio, C₂-C₈-Alkenyloxy, C₂-C₈-Alkinyloxy, C₃-C₇-Cycloalkyl, C₃-C₇-Cycloalkoxy, Mono- und Di-(C₁-C₄-alkyl)-amino und (C₁-C₈-Alkoxy)-carbonyl substituiert ist, bedeuten,
o eine ganze Zahl von 1 bis 5 ist und
p eine ganze Zahl von 1 bis 7 ist,
oder einen monocyclischen oder bicyclischen Heteroarylrest aus der Gruppe Furyl, Thienyl, Pyrrolyl, Pyrazolyl, Thiazolyl, Oxazolyl, Pyridinyl, Pyrimidinyl, Pyrazinyl, Pyridazinyl und Chinolinyl, der jeweils unsubstituiert oder durch einen oder mehrere der genannten Reste U substituiert ist,
R Wasserstoff, C₁-C₁₈-Alkyl, C₃-C₁₂-Cycloalkyl, C₂-C₈-Alkenyl oder C₂-C₈-Alkinyl, Heterocyclyl, das ein 3- bis 7-gliedriger vom Benzol abgeleiteter heterocyclischer Rest ist, in dem mindestens ein CH durch N und/oder mindestens zwei benachbarte CH-Paare durch NH, S und/oder O ersetzt sind und der teilweise oder vollständig hydriert ist, oder Phenyl oder Heteroaryl, ausgewählt aus der Gruppe Pyridyl, Pyrimidyl, Pyridazinyl, Pyrazinyl, Thienyl, Thiazolyl, Oxazolyl, Furyl, Pyrrolyl, Pyrazolyl und Imidazolyl,
wobei jeder der letztgenannten Reste Alkyl, Cycloalkyl, Alkenyl, Alkinyl, Heterocyclyl, Phenyl und Heteroaryl unabhängig voneinander unsubstituiert oder durch einen oder mehrere Reste aus der Gruppe enthaltend Halogen, Cyano, Thio, Nitro, Hydroxy, C₁-C₈-Alkyl, letzteres nur für den Fall cyclischer Reste, C₁-C₈-Haloalkyl, C₁-C₈-Alkoxy, C₂-C₈-Alkenyloxy, C₂-C₈-Alkinyloxy, C₁-C₈-Haloalkoxy, C₁-C₈-Alkylthio, C₂-C₈-Alkenylthio, C₂-C₈-Alkinylthio, C₃-C₇-Cycloalkyl, C₃-C₇-Cycloalkoxy, Reste der Formeln -NR*R** und -CO-NR*R** und -O-CO-NR*R**,
wobei R* und R** in den letztgenannten 3 Resten unabhängig voneinander Wasserstoff, C₁-C₈-Alkyl, C₂-C₈-Alkenyl, C₂-C₈-Alkinyl, Benzyl, Phenyl oder substituiertes Phenyl sind oder gemeinsam mit dem N-Atom einen 3- bis 8-gliedrigen Heterocyclus, der noch bis zu 2 weitere Heteroatome aus der Gruppe N, O und S enthalten und durch C₁-C₄-Alkyl substituiert sein kann, bedeuten,
sowie (C₁-C₈-Alkoxy)-carbonyl, (C₁-C₈-Alkoxy)-thiocarbonyl, (C₂-C₈-Alkenyloxy)-carbonyl, (C₁-C₈-Alkylthio)-carbonyl, (C₂-C₈-Alkenylthio)-carbonyl, (C₂-C₈-Alkinylthio)-carbonyl, (C₂-C₈-Alkinyloxy)-carbonyl, Formyl, (C₁-C₈-Alkyl)-carbonyl, (C₂-C₈-Alkenyl)-carbonyl, (C₂-C₈-Alkinyl)-carbonyl, C₁-C₄-Alkylimino, C₁-C₄-Alkoxyimino, (C₁-C₈-Alkyl)-carbonylamino, (C₂-C₈-Alkenyl)-carbonylamino, (C₂-C₈-Alkinyl)-carbonylamino, (C₁-C₈-Alkoxy)-carbonylamino, (C₂-C₈-Alkenyloxy)-carbonylamino, (C₂-C₈-Alkinyloxy)-carbonylamino, (C₁-C₈-Alkyl)-amino-carbonylamino, (C₁-C₆-Alkyl)-carbonyloxy, das unsubstituiert oder durch Halogen, NO₂, C₁-C₄-Alkoxy oder Phenyl, das unsubstituiert oder ein- oder mehrfach durch Reste aus der Gruppe Halogen, C₁-C₄-Alkyl, C₁-C₄-Alkoxy, C₁-C₄-Halogenalkyl, C₁-C₄-Halogenalkoxy und Nitro substituiert ist, und (C₂-C₆-Alkenyl)-carbonyloxy, (C₂-C₆-Alkinyl)-carbonyloxy, (C₁-C₈-Alkoxy)-carbonyloxy, (C₂-C₈-Alkenyloxy)-carbonyloxy, (C₂-C₈-Alkinyloxy)-carbonyloxy, C₁-C₈-Alkylsulfonyl, Phenyl, Phenyl-C₁-C₆-alkoxy, Phenyl-(C₁-C₆-alkoxy)-carbonyl, Phenoxy, Phenoxy-C₁-C₆-alkoxy, Phenoxy-(C₁-C₆-alkoxy)-carbonyl, Phenoxycarbonyl, Phenylcarbonyloxy, Phenylcarbonylamino, Phenyl-(C₁-C₆-alkyl)-carbonylamino und Phenyl-(C₁-C₆-alkyl)-carbonyloxy,
wobei die letztgenannten 11 Reste im Phenylring unsubstituiert oder durch einen oder mehrere Reste aus der Gruppe Halogen, C₁-C₄-Alkyl, C₁-C₄-Alkoxy, C₁-C₄-Haloalkyl, C₁-C₄-Haloalkoxy und Nitro substituiert sind,
und Reste der Formeln -SiR'₃, -O-SiR'₃, (R')₃Si-C₁-C₆-alkoxy, -CO-O-NR'₂, -O-N=CR'₂, -N=CR'₂, -O-NR'₂, -CH(OR')₂ und -O-(CH₂)ₘ-CH(OR')₂,
worin die R' in den genannten Formeln unabhängig voneinander Wasserstoff, C₁-C₄-Alkyl oder Phenyl, das unsubstituiert oder ein- oder mehrfach durch Reste aus der Gruppe Halogen, C₁-C₄-Alkyl, C₁-C₄-Alkoxy, C₁-C₄-Haloalkyl, C₁-C₄-Haloalkoxy und Nitro substituiert ist, oder paarweise eine C₂-C₆-Alkylenkette und m = 0 bis 6 bedeuten,
und einen substituierten Alkoxyrest der Formel R"O-CHR"'CH(OR")-C₁-C₆-alkoxy,
worin die R" unabhängig voneinander C₁-C₄-Alkyl oder zusammen eine C₁-C₆-Alkylengruppe und R"' Wasserstoff oder C₁-C₄-Alkyl bedeuten,
substituiert ist,
R^{T} einen Rest der Formel -CO-R, -CS-R, -NR^{f}R^{g}, -N=CR^{h}Rⁱ oder SiR^{a}R^{b}R^{c},
wobei R die genannte Bedeutung hat und R^{f}, R^{g}, R^{h} und Rⁱ unabhängig voneinander Wasserstoff, C₁-C₄-Alkyl, C₂-C₄-Alkenyl, C₂-C₄-Alkinyl, Benzyl, Phenyl oder Phenyl, das ein- oder mehrfach durch Reste aus der Gruppe Halogen, C₁-C₄-Alkyl, C₁-C₄-Alkoxy, C₁-C₄-Halogenalkyl, C₁-C₄-Halogenalkoxy und Nitro substituiert ist, sind oder R^{f} und R^{g} gemeinsam mit dem N-Atom einen 5- oder 6-gliedrigen Heterocyclus, der noch bis zu 2 weitere Heteroatome aus der Gruppe N, O und S enthalten und durch C₁-C₄-Alkyl substituiert sein kann, bedeuten und
R^{a}, R^{b} und R^{c} unabhängig voneinander C₁-C₄-Alkyl, C₂-C₄-Alkenyl, C₂-C₄-Alkinyl, Phenyl oder Phenyl, das unsubstituiert oder ein- oder mehrfach durch Reste aus der Gruppe Halogen, C₁-C₄-Alkyl, C₁-C₄-Alkoxy, C₁-C₄-Halogenalkyl, C₁-C₄-Halogenalkoxy und Nitro substituiert ist, sind,
T O, S, NR⁸, N-OR⁸ oder N-O-Acyl, wobei Acyl einen Rest aus der Gruppe Formyl, (C₁-C₄-Alkyl)-carbonyl, Phenylcarbonyl, (C₁-C₄-Alkoxy)-carbonyl, Phenyloxycarbonyl, Benzyloxycarbonyl und C₁-C₄-Alkylsulfonyl bedeutet,
Q O oder S,
q eine ganze Zahl von 0 bis 4,
i eine Laufziffer, welche bei q ungleich 0 alle ganzen Zahlen von 1 bis q annimmt, wobei q die oben angegebene Bedeutung hat,
Xᵢ unabhängig voneinander 0, S, NR⁹, N-(AᵢXᵢ)_{q}-R
Aᵢ unabhängig voneinander unsubstituiertes C₁-C₄-Alkylen, C₂-C₄-Alkenylen oder C₅-C₆-Cycloalkylen und
R⁸, R⁹ unabhängig voneinander H, C₁-C₄-Alkyl, C₂-C₄-Alkenyl, C₂-C₄-Alkinyl, C₃-C₆-Cycloalkyl, C₃-C₆-Cycloalkenyl, Heterocyclyl, das ein 3- bis 7-gliedriger vom Benzol abgeleiteter heterocyclischer Rest ist, in dem mindestens ein CH durch N und/oder mindestens zwei benachbarte CH-Paare durch NH, S und/oder O ersetzt sind und der teilweise oder vollständig hydriert ist, oder Phenyl oder Heteroaryl, ausgewählt aus der Gruppe Pyridyl, Pyrimidyl, Pyridazinyl, Pyrazinyl, Thienyl, Thiazolyl, Oxazolyl, Furyl, Pyrrolyl, Pyrazolyl und Imidazolyl,
bedeuten,
und im Pflanzenschutz übliche Formulierungshilfsmittel enthält.

2. Pflanzenschutzmittel nach Anspruch 1, **dadurch gekennzeichnet, daß** in Formel (I) mindestens einer der Reste R³ und R⁴ einen Rest der Formel 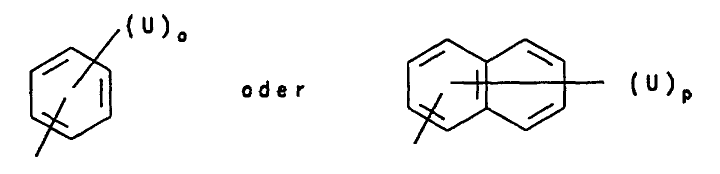 worin
(U) für gleiche oder verschiedene Reste stehen, welche unabhängig voneinander Wasserstoff, Halogen, Cyano, Nitro, Amino, C₁-C₄-Haloalkyl, C₁-C₄-Haloalkoxy, C₁-C₄-Alkyl, C₁-C₄-Alkoxy, Mono-(C₁-C₄-alkyl)-amino, Di-(C₁-C₄-alkyl)-amino, C₁-C₄-Alkylthio oder C₁-C₄-Alkylsulfonyl bedeuten,
o eine ganze Zahl von 1 bis 3 ist und
p eine ganze Zahl von 1 bis 3 ist, oder
R³, R⁴ unabhängig voneinander einen monocyclischen oder bicyclischen Heteroarylrest aus der Gruppe Furyl, Thienyl, Pyrrolyl, Pyrazolyl, Thiazolyl, Oxazolyl, Pyridinyl, Pyrimidinyl, Pyrazinyl, Pyridazinyl und Chinolinyl, der unsubstituiert oder durch ein bis drei der vorstehend genannten Reste U substituiert ist, bedeuten.

3. Pflanzenschutzmittel nach Anspruch 1 oder 2, **dadurch gekennzeichnet, daß** in Formel (I) R³ und R⁴ gleiche oder verschiedene Reste der Formel 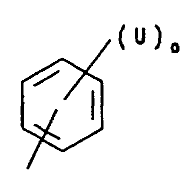 bedeuten, wobei U und o die genannten Bedeutungen haben.

4. Pflanzenschutzmittel nach einem der Ansprüche 1 bis 3, **dadurch gekennzeichnet, daß** in Formel (I)
R Wasserstoff, C₁-C₈-Alkyl, C₄-C₇-Cycloalkyl, C₂-C₈-Alkenyl oder C₂-C₈-Alkinyl, Heterocyclyl, das ein 3- bis 7-gliedriger vom Benzol abgeleiteter heterocyclischer Rest ist, in dem mindestens ein CH durch N und/oder mindestens zwei benachbarte CH-Paare durch NH, S und/oder O ersetzt sind und der teilweise oder vollständig hydriert ist, oder Phenyl oder Heteroaryl, ausgewählt aus der Gruppe Pyridyl, Pyrimidyl, Pyridazinyl, Pyrazinyl, Thienyl, Thiazolyl, Oxazolyl, Furyl, Pyrrolyl, Pyrazolyl und Imidazolyl,
wobei jeder der letztgenannten Reste Alkyl, Cycloalkyl, Alkenyl, Alkinyl, Heterocyclyl, Phenyl und Heteroaryl unabhängig voneinander unsubstituiert oder durch einen oder mehrere Reste aus der Gruppe enthaltend Halogen, Cyano, Thio, Nitro, Hydroxy, C₁-C₄-Alkyl, letzteres nur für den Fall cyclischer Reste, C₁-C₄-Haloalkyl, C₁-C₄-Alkoxy, C₂-C₄-Alkenyloxy, C₂-C₄-Alkinyloxy, C₁-C₄-Haloalkoxy, C₁-C₄-Alkylthio, C₂-C₄-Alkenylthio, C₂-C₄-Alkinylthio, C₅-C₆-Cycloalkyl, C₅-C₆-Cycloalkoxy, Amino, Mono- und Di-(C₁-C₄-alkyl)-amino, (C₁-C₆-Alkoxy)-carbonyl, Reste der Formeln -SiR'₃, -O-NR'₂, -O-N=CR'₂, -N=CR'₂, worin die R' in den genannten Formeln unabhängig voneinander Wasserstoff, C₁-C₂-Alkyl oder Phenyl oder paarweise eine C₂-C₅-Alkylenkette bedeuten, substituiert ist,
bedeutet.

5. Pflanzenschutzmittel nach einem der Ansprüche 1 bis 4, **dadurch gekennzeichnet daß** in Formel (I) R³, R⁴ unabhängig voneinander gleiche oder verschiedene Reste der Formel 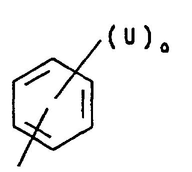 bedeuten und
R Wasserstoff, C₁-C₈-Alkyl, C₄-C₇-Cycloalkyl, C₂-C₈-Alkenyl oder C₂-C₈-Alkinyl, Heterocyclyl, das ein 3- bis 7-gliedriger vom Benzol abgeleiteter heterocyclischer Rest ist, in dem mindestens ein CH durch N und/oder mindestens zwei benachbarte CH-Paare durch NH, S und/oder O ersetzt sind und der teilweise oder vollständig hydriert ist, oder Phenyl oder Heteroaryl, ausgewählt aus der Gruppe Pyridyl, Pyrimidyl, Pyridazinyl, Pyrazinyl, Thienyl, Thiazolyl, Oxazolyl, Furyl, Pyrrolyl, Pyrazolyl und Imidazolyl ist,
wobei jeder der letztgenannten Reste Alkyl, Cycloalkyl, Alkenyl, Alkinyl, Heterocyclyl, Phenyl und Heteroaryl unabhängig voneinander unsubstituiert oder durch einen oder mehrere Reste aus der Gruppe enthaltend Halogen, Cyano, Thio, Nitro, Hydroxy, C₁-C₄-Alkyl, letzteres nur für den Fall cyclischer Reste, C₁-C₄-Haloalkyl, C₁-C₄-Alkoxy, C₂-C₄-Alkenyloxy, C₂-C₄-Alkinyloxy, C₁-C₄-Haloalkoxy, C₁-C₄-Alkylthio, C₂-C₄-Alkenylthio, C₂-C₄-Alkinylthio, C₅-C₆-Cycloalkyl, C₅-C₆-Cycloalkoxy, Amino, Mono- und Di-(C₁-C₄-alkyl)-amino, (C₁-C₆-Alkoxy)-carbonyl, Reste der Formeln -SiR'₃, -O-NR'₂, -O-N=CR'₂, -N=CR'₂, worin die R' in den genannten Formeln unabhängig voneinander Wasserstoff, C₁-C₂-Alkyl oder Phenyl oder paarweise eine C₂-C₅-Alkylenkette bedeuten, substituiert ist, bedeutet und
R^{T} einen Rest der Formel -CO-R, -NR^{f}R^{g} oder -N = CR^{h}Rⁱ bedeutet.

6. Pflanzenschutzmittel nach einem der Ansprüche 1 bis 5, **dadurch gekennzeichnet daß** in Formel (I)
R Wasserstoff, C₁-C₈-Alkyl, C₅-C₆-Cycloalkyl, C₂-C₈-Alkenyl oder C₂-C₈-Alkinyl bedeutet, wobei jeder der letztgenannten 4 Reste unabhängig voneinander unsubstituiert oder durch einen oder mehrere Reste aus der Gruppe enthaltend Halogen, Cyano, Nitro, C₁-C₄-Alkoxy, C₂-C₄-Alkenyloxy, C₂-C₄-Alkinyloxy, C₅-C₆-Cycloalkyl, C₅-C₆-Cycloalkoxy, Mono- und Di-(C₁-C₄-alkyl)-amino, Reste der Formeln -SiR'₃, -O-N=CR'₂, -N=CR'₂, worin die R' in den genannten Formeln unabhängig voneinander Wasserstoff, C₁-C₂-Alkyl oder Phenyl oder paarweise eine C₂-C₅-Alkylenkette bedeuten, substituiert ist.

7. Pflanzenschutzmittel nach einem der Ansprüche 1 bis 6, **dadurch gekennzeichnet daß** in Formel (I)
R¹ einen Rest der Formel 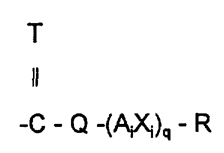
und
T O oder NR⁸,
Q O,
q eine ganze Zahl von 0 bis 4,
i eine Laufziffer, welche bei q ungleich 0 alle ganzen Zahlen von 1 bis q annimmt, wobei q die oben angegebene Bedeutung hat,
Xᵢ unabhängig voneinander O, S, NR⁹, N-(AᵢXᵢ)_{q}-R
Aᵢ unabhängig voneinander C₁-C₄-Alkylen,
R⁸, R⁹ unabhängig voneinander H, C₁-C₄-Alkyl, C₂-C₄-Alkenyl, C₂-C₄-Alkinyl oder C₅-C₆-Cycloalkyl
bedeuten.

8. Pflanzenschutzmittel, **dadurch gekennzeichnet, daß** es mindestens ein Pestizid sowie als Safener mindestens eine Verbindung der Formel (I) oder deren Salze nach einem der Ansprüche 1 bis 7 enthält.

9. Verbindungen der Formel (I) und deren Salze, wie sie nach einem der Ansprüche 1 bis 7 definiert sind, ausgenommen Verbindung 3-Carbethoxy-5-methyl-5-vinyl-isoxazolin.

10. Verfahren zur Herstellung von Verbindungen der Formel (I) oder deren Salzen, wie sie nach Anspruch 9 definiert sind, **dadurch gekennzeichnet, daß** man eine Verbindung der Formel (II),
R³R⁴C = CHR² (II)
worin R², R³ und R⁴ wie in der Verbindung der Formel (I) definiert sind, mit einem Nitriloxid der Formel (III)
⁽⁻⁾O - N = ⁽⁺⁾C - R¹ (III)
worin R¹ wie in Formel (I) definiert ist, umsetzt.

11. Verfahren zum Schutz von Kulturpflanzen vor phytotoxischen Nebenwirkungen von Pflanzenschutzmittel-Wirkstoffen (Pestiziden), **dadurch gekennzeichnet, daß** eine wirksame Menge von mindestens einer der Verbindungen der Formel (I) oder deren Salzen nach einem der Ansprüche 1 bis 7 vor, nach oder gleichzeitig mit dem jeweiligen Pestizid auf die Pflanzen, Pflanzensamen oder die Anbaufläche appliziert wird.

12. Verwendung von Verbindungen der Formel (I) oder deren Salzen, wie sie nach einem der Ansprüche 1 bis 7 definiert sind, als Safener zum Schützen von Kulturpflanzen vor phytotoxischen Nebenwirkungen von Pflanzenschutzmittel-Wirkstoffen (Pestiziden).

## Claims

1. A crop protection composition, which comprises, as crop-plant-protecting component, a compound of the formula (I) or a salt thereof 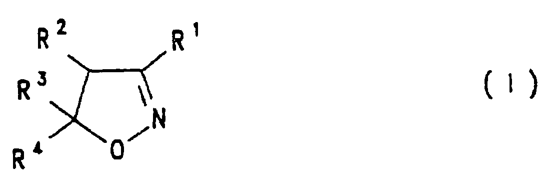 in which
R¹ is an acyl radical selected from the group of the radicals of the formulae 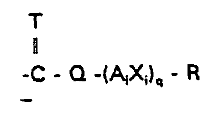 and 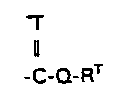 in which R, R^{T}, T, Q, Aᵢ, Xᵢ and q are as defined further below,
R² is hydrogen,
R³ and R⁴ independently of one another are C₁-C₁₈-alkyl, C₃-C₁₂-cycloalkyl, C₂-C₈-alkenye or C₂-C₈-alkynyl or a radical of the formula 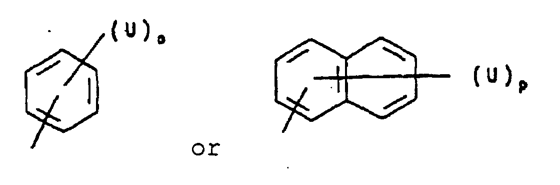 in which
(U) are identical or different radicals which, independently of one another, are hydrogen, halogen, cyano, nitro, amino or C₁-C₈-haloalkyl, C₁-C₈-haloalkoxy, C₁-C₈-alkyl, C₁-C₈-alkoxy, mono (C₁-C₄-alkyl) amino, di (C₁-C₄-alkyl)amino, C₁-C₈-alkylthio or C₁-C₈-alkylsulfonyl, where each of the 8 lastmentioned radicals is unsubstituted or substituted by one or more identical or different substituents selected from the group consisting of halogen, C₁-C₈-haloalkoxy, nitro, cyano, hydroxyl, C₁-C₈-alkoxy and a C₁-C₈-alkoxy group in which one or more CH₂ groups are replaced by oxygen, and C₁-C₈-alkylthio, C₁-C₆-alkylsulfinyl, C₁-C₆-alkylsulfonyl, C₂-C₈-alkenylthio, C₂-C₈-alkynylthio, C₂-C₈-alkenyloxy, C₂-C₈-alkynyloxy, C₃-C₇-cycloalkyl, C₃-C₇-cycloalkoxy, mono- and di (C₁-C₄-alkyl) -amino and (C₁-C₈-alkoxy)carbonyl,
o is an integer from 1 to 5 and
p is an integer from 1 to 7,
or a monocyclic or bicyclic heteroaryl radical selected from the group consisting of furyl, thienyl, pyrrolyl, pyrazolyl, thiazolyl, oxazolyl, pyridinyl, pyrimidinyl, pyrazinyl, pyridazinyl and quinolinyl which is in each case unsubstituted or substituted by one or more of the radicals U mentioned,
R is hydrogen, C₁-C₁₈-alkyl, C₃-C₁₂-cycloalkyl, C₂-C₈-alkenyl or C₂-C₈-alkynyl, heterocyclyl, which is a 3- to 7-membered heterocyclic radical derived from benzene in which at least one CH is replaced by N and/or at least two adjacent CH pairs are replaced by NH, S and/or O and which is partially or fully hydrogenated, or is phenyl or heteroaryl selected from the group consisting of pyridyl, pyrimidyl, pyridazinyl, pyrazinyl, thienyl, thiazolyl, oxazolyl, furyl, pyrrolyl, pyrazolyl and imidazolyl,
where each of the lastmentioned radicals alkyl, cycloalkyl, alkenyl, alkynyl, heterocyclyl, phenyl and heteroaryl, independently of the others, is unsubstituted or substituted by one or more radicals selected from the group consisting of halogen, cyano, thio, nitro, hydroxyl, C₁-C₈-alkyl, the latter only in the case of cyclic radicals, C₁-C₈-haloalkyl, C₁-C₈-alkoxy, C₂-C₈-alkenyloxy, C₂-C₈-alkynyloxy, C₁-C₈-haloalkoxy, C₁-C₈-alkylthio, C₂-C₈-alkenylthio, C₂-C₈-alkynylthio, C₃-C₇-cycloalkyl, C₃-C₇-cycloalkoxy, radicals of the formulae -NR*R** and -CO-NR*R** and -O-CO-NR*R**,
where R* and R** in the 3 lastmentioned radicals independently of one another are hydrogen, C₁-C₈-alkyl, C₂-C₈-alkenyl, C₂-C₈-alkynyl, benzyl, phenyl or substituted phenyl or together with the nitrogen atom are a 3- to 8-membered heterocycle which may contain up to 2 further heteroatoms from the group consisting of N, O and S and which may be substituted by C₁-C₄-alkyl,
and also (C₁-C₈-alkoxy) carbonyl, (C₁-C₈-alkoxy)thiocarbonyl, (C₂-C₈-alkenyloxy)-carbonyl, (C₁-C₈-alkylthio)carbonyl, (C₂-C₈-alkenylthio)carbonyl, (C₂-C₈-alkynylthio)-carbonyl, (C₂-C₈-alkynyloxy)carbonyl, formyl, (C₁-C₈-alkyl)carbonyl, (C₂-C₈-alkenyl)carbonyl, (C₂-C₈-alkynyl)carbonyl, C₁-C₄-alkylimino, C₁-C₄-alkoxyimino, (C₁-C₈-alkyl)carbonylamino, (C₂-C₈-alkenyl)carbonylamino, (C₂-C₈-alkynyl)-carbonylamino, (C₁-C₈-alkoxy)carbonylamino, (C₂-C₈-alkenyloxy) carbonylamino, (C₂-C₈-alkynyloxy) carbonylamino, (C₁-C₈-alkyl) aminocarbonylamino, (C₁-C₈-alkyl)carbonyloxy, which is unsubstituted or substituted by halogen, NO₂, C₁-C₄-alkoxy or phenyl, which is unsubstituted or mono- or polysubstituted by radicals selected from the group consisting of halogen, C₁-C₄-alkyl, C₁-C₄-alkoxy, C₁-C₄-haloalkyl, C₁-C₄-haloalkoxy and nitro, and (C₂-C₆-alkenyl) carbonyloxy, (C₂-C₆-alkynyl)-carbonyloxy, (C₁-C₈-alkoxy)carbonyloxy, (C₂-C₈-alkenyloxy)carbonyloxy, (C₂-C₈-alkynyloxy)-carbonyloxy, C₁-C₈-alkylsulfonyl, phenyl, phenyl-C₁-C₆-alkoxy, phenyl (C₁-C₆-alkoxy)-carbonyl, phenoxy, phenoxy-C₁-C₆-alkoxy, phenoxy (C₁-C₆-alkoxy) carbonyl, phenoxycarbonyl, phenylcarbonyloxy, phenylcarbonylamino, phenyl (C₁-C₆-alkyl) carbonylamino and phenyl (C₁-C₆-alkyl) carbonyloxy,
where the 11 lastmentioned radicals are unsubstituted in the phenyl ring or substituted by one or more radicals selected from the group consisting of halogen, C₁-C₄-alkyl, C₁-C₄-alkoxy, C₁-C₄-haloalkyl, C₁-C₄-haloalkoxy and nitro,
and radicals of the formulae -SiR'₃, -O-SiR'₃, (R')₃Si-C₁-C₆-alkoxy, -CO-O-NR'₂, -O-N=CR'₂, -N=CR'₂, -O-NR'₂, -CH(OR')₂ and -O-(CH₂)ₘ-CH(OR')₂,
in which R' in the formulae mentioned, independently of one another, are hydrogen, C₁-C₄-alkyl or phenyl, which is unsubstituted or mono- or polysubstituted by radicals selected from the group consisting of halogen, C₁-C₄-alkyl, C₁-C₄-alkoxy, C₁-C₄-haloalkyl, C₁-C₄-haloalkoxy and nitro, or a pair of R' is a C₂-C₆-alkylene chain, and m = 0 to 6,
and a substituted alkoxy radical of the formula R' 'O-CHR'''CH(OR")-C₁-C₆-alkoxy,
in which R" independently of one another are C₁-C₄-alkyl or together are a C₁-C₆-alkylene group and R''' is hydrogen or C₁-C₄-alkyl,
R^{T} is a radical of the formula -CO-R, -CS-R, -NR^{f}R^{g}, -N=CR^{h}Rⁱ or SiR^{a}R^{b}R^{c}, where R is as defined above and R^{f}, R^{g}, R^{h} and Rⁱ independently of one another are hydrogen, C₁-C₄-alkyl, C₂-C₄-alkenyl, C₂-C₄-alkynyl, benzyl, phenyl or phenyl which is mono- or polysubstituted by radicals selected from the group consisting of halogen, C₁-C₄-alkyl, C₁-C₄-alkoxy, C₁-C₄-haloalkyl, C₁-C₄-haloalkoxy and nitro, or R^{f} and R^{g} together with the nitrogen atom are a 5- or 6-membered heterocycle which may contain up to 2 further heteroatoms selected from the group consisting of N, O and S and which may be substituted by C₁-C₄-alkyl, and R^{a}, R^{b} and R^{c} independently of one another are C₁-C₄-alkyl, C₂-C₄-alkenyl, C₂-C₄-alkynyl, phenyl or phenyl which is unsubstituted or mono- or polysubstituted by radicals selected from the group consisting of halogen, C₁-C₄-alkyl, C₁-C₄-alkoxy, C₁-C₄-haloalkyl, C₁-C₄-haloalkoxy and nitro,
T is O, S, NR⁸, N-OR⁸ or N-O-acyl, where acyl is a radical selected from the group consisting of formyl, (C₁-C₄-alkyl)carbonyl, phenylcarbonyl, (C₁-C₄)-alkoxy)carbonyl, phenyloxycarbonyl, benzyloxycarbonyl and C₁-C₄-alkylsulfonyl,
Q is O or S,
q is an integer from 0 to 4,
i is a running number which, if q is not 0, adopts the value of all integers from 1 to q, q being defined as above,
Xᵢ independently of one another are O, S, NR⁹, N-(AᵢXᵢ)_{q}-R
Aᵢ independently of one another are unsubstituted C₁-C₄-alkylene, C₂-C₄-alkenylene or C₅-C₆-cycloalkylene and
R⁸,R⁹ independently of one another are H, C₁-C₄-alkyl, C₂-C₄-alkenyl, C₂-C₄-alkynyl, C₃-C₆-cycloalkyl, C₃-C₆-cycloalkenyl, heterocyclyl, which is a 3- to 7-membered heterocyclic radical derived from benzene in which at least one CH is replaced by N and/or at least two adjacent CH pairs are replaced by NH, S and/or O and which is partially or fully hydrogenated, or phenyl or heteroaryl selected from the group consisting of pyridyl, pyrimidyl, pyridazinyl, pyrazinyl, thienyl, thiazolyl, oxazolyl, furyl, pyrrolyl, pyrazolyl and imidazolyl,
and formulation auxiliaries which are customary in crop protection.

2. The crop protection composition as claimed in claim 1 wherein in formula (I) at least one of the radicals R³ and R⁴ is a radical of the formula 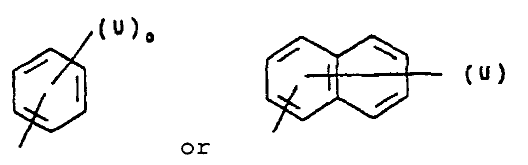 in which
(U) are identical or different radicals which, independently of one another, are hydrogen, halogen, cyano, nitro, amino, C₁-C₄-haloalkyl, C₁-C₄-haloalkoxy, C₁-C₄-alkyl, C₁-C₄-alkoxy, mono (C₁-C₄-alkyl) amino, di (C₁-C₄-alkyl) amino, C₁-C₄-alkylthio or C₁-C₄-alkylsulfonyl,
O is an integer from 1 to 3 and
P is an integer from 1 to 3, or
R³,R⁴ independently of one another are a monocyclic or bicyclic heteroaryl radical selected from the group consisting of furyl, thienyl, pyrrolyl, pyrazolyl, thiazolyl, oxazolyl, pyridinyl, pyrimidinyl, pyrazinyl, pyridazinyl and quinolinyl, unsubstituted or substituted by one to three of the radicals U mentioned above.

3. The crop protection composition as claimed in claim 1 or 2, wherein in formula (I) R³ and R⁴ are identical or different radicals of the formula 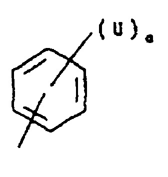 where U and o are as defined above.

4. The crop protection composition as claimed in any of claims 1 to 3, wherein in formula (I)
R is hydrogen, C₁-C₈-alkyl, C₄-C₇-cycloalkyl, C₂-C₈-alkenyl or C₂-C₈-alkynyl, heterocyclyl, which is a 3- to 7-membered heterocyclic radical derived from benzene in which at least one CH is replaced by N and/or at least two adjacent CH pairs are replaced by NH, S and/or O and which is partially or fully hydrogenated, or phenyl or heteroaryl selected from the group consisting of pyridyl, pyrimidyl, pyridazinyl, pyrazinyl, thienyl, thiazolyl, oxazolyl, furyl, pyrrolyl, pyrazolyl and imidazolyl,
where each of the lastmentioned radicals alkyl, cycloalkyl, alkenyl, alkynyl, heterocyclyl, phenyl and heteroaryl, independently of the others, is unsubstituted or substituted by one or more radicals selected from the group consisting of halogen, cyano, thio, nitro, hydroxyl, C₁-C₄-alkyl, the latter only in the case of cyclic radicals, C₁-C₄-haloalkyl, C₁-C₄-alkoxy, C₂-C₄-alkenyloxy, C₂-C₄-alkynyloxy, C₁-C₄-haloalkoxy, C₁-C₄-alkylthio, C₂-C₄-alkenylthio, C₂-C₄-alkynylthio, C₅-C₆-cycloalkyl, C₅-C₆-cycloalkoxy, amino, mono- and di(C₁-C₄-alkyl)-amino, (C₁-C₆-alkoxy)carbonyl, radicals of the formulae -SiR'₃, -O-NR'₂, -O-N=CR'₂, -N=CR'₂, in which the R' in the formulae mentioned, independently of one another, are hydrogen, C₁-C₂-alkyl or phenyl, or a pair of R' is a C₂-C₅-alkylene chain.

5. The crop protection composition as claimed in any of claims 1 to 4, wherein in formula (I)
R³, R⁴ independently of one another are identical or different radicals of the formula 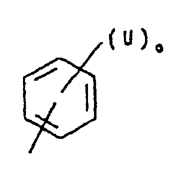 and
R is hydrogen, C₁-C₈-alkyl, C₄-C₇-cycloalkyl, C₂-C₈-alkenyl or C₂-C₈-alkynyl, heterocyclyl, which is a 3- to 7-membered heterocyclic radical derived from benzene in which at least one CH is replaced by N and/or at least two adjacent CH pairs are replaced by NH, S and/or O and which is partially or fully hydrogenated, or phenyl or heteroaryl selected from the group consisting of pyridyl, pyrimidyl, pyridazinyl, pyrazinyl, thienyl, thiazolyl, oxazolyl, furyl, pyrrolyl, pyrazolyl and imidazolyl,
where each of the lastmentioned radicals alkyl, cycloalkyl, alkenyl, alkynyl, heterocyclyl, phenyl and heteroaryl, independently of the others, is unsubstituted or substituted by one or more radicals selected from the group consisting of halogen, cyano, thio, nitro, hydroxyl, C₁-C₄-alkyl, the latter only in the case of cyclic radicals, C₁-C₄-haloalkyl, C₁-C₄-alkoxy, C₂-C₄-alkenyloxy, C₂-C₄-alkynyloxy, C₁-C₄-haloalkoxy, C₁-C₄-alkylthio, C₂-C₄-alkenylthio, C₂-C₄-alkynylthio, C₅-C₆-cycloalkyl, C₅-C₆-cycloalkoxy, amino, mono- and di (C₁-C₄-alkyl)amino, (C₁-C₆-alkoxy)carbonyl, radicals of the formulae -SiR'₃, -O-NR'₂, -O-N=CR'₂, -N=CR'₂, in which the R' in the formulae mentioned, independently of one another, are hydrogen, C₁-C₂-alkyl or phenyl, or a pair of R' is a C₂-C₅-alkylene chain,
R^{T} is a radical of the formula -CO-R, -NR^{f}R^{g} or -N=CR^{h}Rⁱ.

6. The crop protection composition as claimed in any of claims 1 to 5, wherein in formula (I)
R is hydrogen, C₁-C₈-alkyl, C₅-C₆-cycloalkyl, C₂-C₈-alkenyl or C₂-C₈-alkynyl, where each of the 4 lastmentioned radicals independently of the others is unsubstituted or substituted by one or more radicals selected from the group consisting of halogen, cyano, nitro, C₁-C₄-alkoxy, C₂-C₄-alkenyloxy, C₂-C₄-alkynyloxy, C₅-C₆-cycloalkyl, C₅-C₆-cycloalkoxy, mono- and di (C₁-C₄-alkyl)amino, radicals of the formulae -SiR'₃, -O-N=CR'₂, -N=CR'₂, in which the R' in the formulae mentioned independently of one another are hydrogen, C₁-C₂-alkyl or phenyl, or a pair of R' are a C₂-C₅-alkylene chain.

7. The crop protection composition as claimed in any of claims 1 to 6, wherein in formula (I)
R¹ is a radical of the formula 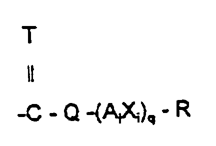 and
T is O or NR⁸,
Q is O,
q is an integer from 0 to 4,
i is a running number which, if q is not 0, adopts the value of all integers from 1 to q, q being defined as above,
Xᵢ independently of one another are O, S, NR⁹, N-(AᵢXᵢ)_{q}-R
Aᵢ independently of one another are C₁-C₄-alkylene,
R⁸, R⁹ independently of one another are H, C₁-C₄-alkyl, C₂-C₄-alkenyl, C₂-C₄-alkynyl or C₅-C₆-cycloalkyl.

8. A crop protection composition, which comprises at least one pesticide and, as safener, at least one compound of the formula (I) or a salt thereof as claimed in any of claims 1 to 7.

9. A compound of the formula (I) or a salt thereof as defined in any of claims 1 to 7, except for the compound 3-carbethoxy-5-methyl-5-vinylisoxazoline.

10. A process for preparing compounds of the formula (I) or salts thereof as defined in claim 9, which comprises reacting a compound of the formula (II)
R³R⁴C = CHR² (II)
in which R², R³ and R⁴ are as defined in the compound of the formula (I), with a nitrile oxide of the formula (III)
⁽⁻⁾O - N = ⁽⁺⁾C - R¹ (III)
in which R¹ is as defined in formula (I).

11. A method for protecting crop plants against the phytotoxic side effects of the active compounds (pesticides) in crop protection compositions, which comprises applying an effective amount of at least one of the compounds of the formula (I) or a salt thereof as claimed in any of claims 1 to 7 prior to, after or simultaneously with the pesticide in question onto the plants, plant seeds or the area under cultivation.

12. The use of compounds of the formula (I) or salts thereof as defined in any of claims 1 to 7 as safeners for protecting crop plants against the phytotoxic side effects of the active compounds (pesticides) in crop protection compositions.

## Revendications

1. Agent phytoprotecteur **caractérisé en ce qu'**il contient en tant que constituant phytoprotecteur un composé de formule (I) ou son sel 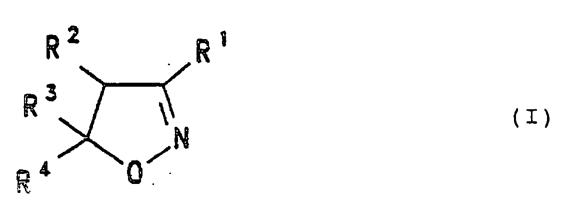 où
R¹ représente un reste acyle pris dans le groupe des restes de formule 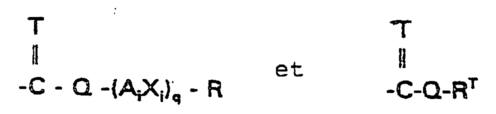 où R, R^{T}, T, Q, Aᵢ, Xᵢ et q sont définis comme donné ci-après,
R² représente un atome d'hydrogène,
R³ et R⁴ représentent, indépendamment l'un de l'autre, des groupes alkyle en C₁-C₁₈, cycloalkyle en C₃-C₁₂, alcényle en C₂-C₈ ou alcynyle en C₂-C₈ ou un reste de formule 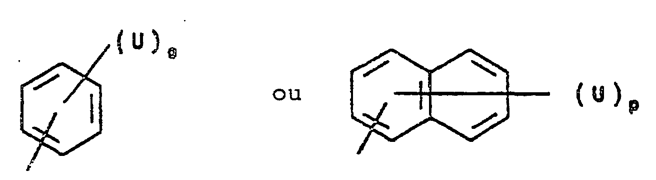 où
(U) représente des restes identiques ou différents qui représentent indépendamment les uns des autres des atomes d'hydrogène, des atomes d'halogène, des groupes cyano, nitro, amino ou haloalkyle en C₁-C₈, haloalkoxy en C₁-C₈, alkyle en C₁-C₈, alkoxy en C₁-C₈, mono-(alkyl en C₁-C₄)amino, di-(alkyl en C₁-C₄)amino, (alkyl en C₁-C₈)thio ou (alkyl en C₁-C₈)sulfonyle, chacun des 8 derniers restes cités étant non substitué ou substitué par un ou plusieurs substituants identiques ou différents pris dans le groupe des substituants halogène, haloalkoxy en C₁-C₈, nitro, cyano, hydroxy, alkoxy en C₁-C₈ et un groupe alkoxy en C₁-C₈ où un ou plusieurs groupes CH₂ sont remplacés par des atomes d'oxygène, et (alkyl en C₁-C₈)thio, (alkyl en C₁-C₆)-sulfinyle, (alkyl en C₁-C₆)-sulfonyle, (alcényle en C₂-C₈)thio, (alcynyle en C₂-C₈)thio, alcényloxy en C₂-C₈, alcynyloxy en C₂-C₈, cycloalkyle en C₃-C₇, cycloalkoxy en C₃-C₇, mono- et di-(alkyl en C₁-C₄)amino et (alkoxy en C₁-C₈)-carbonyle,
O est un entier de 1 à 5 et
p est un entier de 1 à 7,
ou un reste hétéroaryle monocyclique ou bicyclique pris dans le groupe des restes furyle, thiényle, pyrrolyle, pyrazolyle, thiazolyle, oxazolyle, pyridinyle, pyrimidinyle, pyrazinyle, pyridazinyle et quinoléinyle, chacun étant non substitué ou substitué par un ou plusieurs des restes U précités,
R représente un atome d'hydrogène, des groupes alkyle en C₁-C₁₈, cycloalkyle en C₃-C₁₂, alcényle en C₂-C₈ ou alcynyle en C₂-C₈, hétérocyclyle, qui est un reste hétérocyclique de 3 à 7 chaînons dérivant du benzène, dans lequel au moins un groupe CN est remplacé par un atome de N et/ou au moins deux paires CH adjacents sont remplacés par des groupes NH, S et/ou O et qui est partiellement ou complètement hydrogéné, ou phényle ou hétéroaryle, pris dans le groupe des restes pyridyle, pyrimidyle, pyridazinyle, pyrazinyle, thiényle, thiazolyle, oxazolyle, furyle, pyrrolyle, pyrazolyle et imidazolyle,
chacun des derniers restes cités alkyle, cycloalkyle, alcényle, alcynyle, hétérocyclyle, phényle et hétéroaryle peut être, indépendamment, non substitué ou substitué par un ou plusieurs restes pris dans le groupe comportant des atomes d'halogènes, des groupes cyano, thio, nitro, hydroxy, alkyle en C₁-C₈, ce dernier seulement pour le cas de restes cycliques, haloalkyle en C₁-C₈, alkoxy en C₁-C₈, alcényloxy en C₂-C₈, alcynyloxy en C₂-C₈, haloalkoxy en C₁-C₈, (alkyl en C₁-C₈)thio, (alcényle en C2-C8)-thio, (alcynyle en C₂-C₈)thio, cycloalkyle en C₃-C₇, cycloalkoxy en C₃-C₇, restes de formules -NR*R** et -CO-NR*R** et -O-CO-NR*R**,
où R* et R** dans les trois derniers restes cités représentent, indépendamment l'un de l'autre, des atomes d'hydrogène, des groupes alkyle en C₁-C₈, alcényle en C₂-C₈, alcynyle en C₂-C₈, benzyle, phényle ou phényle substitué ou ensemble avec l'atome de N forment un hétérocycle de 3 à 8 chaînons pouvant contenir encore jusqu'à 2 autres hétéroatomes pris parmi les atomes de N, de O et de S et être substitué par un substituant alkyle en C₁-C₄,
ainsi que (alkoxy en C₁-C₈)carbonyle, (alkoxy en C₁-C₈)thiocarbonyle, (alcényloxy en C₂-C₈)carbonyle, (alkylthio en C₁-C₈)-carbonyle, (alcényle-thio en C₂-C₈)-carbonyle, (alcynyle-thio en C₂-C₈)-carbonyle, (alcynyloxy en C₂-C₈)carbonyle, formyle, (alkyl en C₁-C₈)carbonyle, (alcényle en C₂-C₈)carbonyle, (alcynyle en C₂-C₈)carbonyle, (alkyl en C₁-C₄)imino, (alkoxy en C₁-C₄)imino, (alkyl en C₁-C₈)-carbonylamino, (alcényle en C₂-C₈)-carbonylamino, (alcynyle en C₂-C₈)-carbonylamino, (alkoxy en C₁-C₈)-carbonylamino, (alcényloxy en C₂-C₈)-carbonylamino, (alcynyloxy en C₂-C₈)-carbonylamino, (alkyl en C₁-C₈)-aminocarbonylamino, (alkyl en C₁-C₆)carbonyloxy qui est non substitué ou substitué par des atomes d'halogènes, des groupes NO₂, alkoxy en C₁-C₄ ou phényle lequel est éventuellement non substitué ou substitué une ou plusieurs fois par des restes pris dans le groupe comprenant des atomes d'halogènes, des groupes alkyle en C₁-C₄, alkoxy en C₁-C₄, haloalkyle en C₁-C₄, haloalkoxy en C₁-C₄ et nitro, et (alcényle en C₂-C₆)-carbonyloxy, (alcynyle en C₂-C₆)-carbonyloxy, (alkoxy en C₁-C₈)-carbonyloxy, (alcényloxy en C₂-C₈)-carbonyloxy, (alcynyloxy en C₂-C₈)-carbonyloxy, (alkyl en C₁-C₈)sulfonyle, phényle, phénylalkoxy en C₁-C₆, phényl(alkoxy en C₁-C₆)carbonyls, phénoxy, phénoxyalkoxy en C₁-C₆, phénoxy-(alkoxy en C₁-C₆)carbonyle, phénoxycarbonyle, phénylcarbonyloxy, phénylcarbonylamino, phényl(alkyl en C₁-C₆)-carbonylamino et phényl(alkyl en C₁-C₆)-carbonyloxy,
les 11 derniers restes cités pouvant être non substitués ou substitués sur le cycle phényle par un ou plusieurs restes pris dans le groupe des restes halogène, alkyle en C₁-C₄, alkoxy en C₁-C₄, haloalkyle en C₁-C₄, haloalkoxy en C₁-C₄ et nitro,
et des restes de formules -SiR'₃, -O-SiR'₃, (R')₃Si-alkoxy en C₁-C₆, -CO-O-NR'₂, -O-N=CR'₂, -N=CR'₂, -O-NR'₂, -CH(OR')₂ et -O-(CH₂)ₘ-CH(OR')₂,
où les restes R' dans les formules citées représentent, indépendamment les uns des autres, des atomes d'hydrogène, des groupes alkyle en C₁-C₄ ou phényle, qui est non substitué ou substitué une ou plusieurs fois par des restes pris dans le groupe des restes halogène, alkyle en C₁-C₄, alkoxy en C₁-C₄, haloalkyle en C₁-C₄, haloalkoxy en C₁-C₄ et nitro, ou par paires, représentent une chaîne alkylène en C₂-C₆ et m = 0 à 6,
et un reste alkoxy substitué de formule R' 'O-CHR' ' 'CH(OR' ')-alkoxy en C₁-C₆,
où les restes R' ' représentent, indépendamment les uns des autres, des groupes alkyle en C₁-C₄ ou ensemble un groupe alkylène en C₁-C₆ et R''' représente un atome d'hydrogène ou un groupe alkyle en C₁-C₄,
R^{T} représente un reste de formule -CO-R, -CS-R, -NR^{f}R^{g}, -N=CR^{h}Rⁱ ou SiR^{a}R^{b}R^{c},
R possédant la signification donnée et R^{f}, R^{g}, R^{h} et Rⁱ représentent, indépendamment les uns des autres, des atomes d'hydrogène, des groupes alkyle en C₁-C₄, alcényle en C₂-C₄, alcynyle en C₂-C₄, benzyle, phényle ou phényle lequel est substitué une ou plusieurs fois par des restes pris dans le groupe des restes halogène, alkyle en C₁-C₄, alkoxy en C₁-C₄, haloalkyle en C₁-C₄, haloalkoxy en C₁-C₄ et nitro, ou R^{f} et R^{g} forment conjointement avec l'atome de N un hétérocycle à 5 ou 6 chaînons, lequel peut comporter encore jusqu'à 2 autres hétéroatomes pris parmi les atomes de N, de O et de S et peut être substitué par un substituant alkyle en C₁-C₄, et R^{a}, R^{b} et R^{c} représentent, indépendamment les uns des autres, des groupes alkyle en C₁-C₄, alcényle en C₂-C₄, alcynyle en C₂-C₄, phényle ou phényle substitué,
T représente des groupes O, S, NR⁸, N-OR⁸ ou N-O-acyle, acyle représentant un reste pris dans le groupe des restes formyle, (alkyl en C₁-C₄)-carbonyle, phénylcarbonyle, (alkoxy en C₁-C₄)-carbonyle, phénoxycarbonyle, benzyloxycarbonyle et (alkyl en C₁-C₄)sulfonyle,
Q représente des atomes de O ou de S,
q est un entier de 0 à 4,
i est un nombre qui, pour q inégal à zéro, prend tous les entiers de 1 à q, q ayant la signification donnée ci-dessus,
Xᵢ représentent, indépendamment les uns des autres, des groupes O, S, NR^{g}, N-(AᵢXᵢ)_{q}-R,
Aᵢ représentent, indépendamment les uns des autres, des groupes alkylène en C₁-C₄, alcénylène en C₂-C₄ ou cycloalkylène en C₅-C₈, et
R⁸, R⁹ représentent, indépendamment l'un de l'autre, des atomes d'hydrogène, des groupes alkyle en C₁-C₄, alcényle en C₂-C₄, alcynyle en C₂-C₄, cycloalkyle en C₃-C₆, cycloalcényle en C₃-C₆, hétérocyclyle, qui est un reste hétérocyclique de 3 à 7 chaînons dérivant du benzène, dans lequel au moins un groupe CH est remplacé par un atome de N et/ou au moins deux paires CH adjacents sont remplacés par des groupes NH, S et/ou O, et sont partiellement ou entièrement hydrogénés, ou représentent phényle ou hétéroaryle, pris dans le groupe comportant des restes pyridyle, pyrimidyle, pyridazinyle, pyrazinyle, thiényle, thiazolyle, oxazolyle, furyle, pyrrolyle, pyrazolyle et imidazolyle,
et des adjuvants de formulation usuels dans la protection de végétaux.

2. Agent phytoprotecteur selon la revendication 1, **caractérisé en ce que** dans la formule (I), au moins un des restes R³ et R⁴ représente un reste de formule 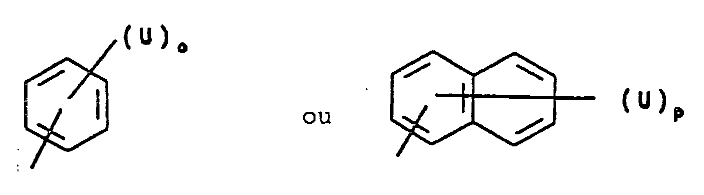
(U) représente des restes identiques ou différents qui représentent, indépendamment les uns des autres, des atomes d'hydrogène, des atomes d'halogène, des groupes cyano, nitro, amino, haloalkyle en C₁-C₄, haloalkoxy en C₁-C₄, alkyle en C₁-C₄, alkoxy en C₁-C₄, mono(alkyl en C₁-C₄) amino, di-(alkyl en C₁-C₄)amino, (alkyl en C₁-C₄)-thio ou (alkyl en C₁-C₄)sulfonyle
O est un entier de 1 à 3, et
p est un entier de 1 à 3, ou
R³, R⁴ représentent, indépendamment l'un de l'autre, un reste hétéroaryle monocyclique ou bicyclique pris dans le groupe des restes furyle, thiényle, pyrrolyle, pyrazolyle, thiazolyle, oxazolyle, pyridinyle, pyrimidinyle, pyrazinyle, pyridazinyle et quinoléinyle, chacun étant non substitué ou substitué par un à trois des restes U précités.

3. Agent phytoprotecteur selon la revendication 1 ou 2, **caractérisé en ce que** dans la formule (I), au moins un des restes R³ et R⁴ représente un reste de formule 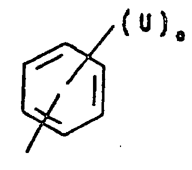
U et o possédant les significations précitées.

4. Agent phytoprotecteur selon l'une des revendications 1 à 3, **caractérisé en ce que** dans la formule (I),
R représente un atome d'hydrogène, des groupes alkyle en C₁-C₈, cycloalkyle en C₄-C₇, alcényle en C₂-C₈ ou alcynyle en C₂-C₈, hétérocyclyle, qui est un reste de 3 à 7 chaînons dérivant du benzène, dans lequel au moins un groupe CH est remplacé par un atome de N et/ou au moins deux paires adjacents sont remplacés par des groupes NH, S et/ou O et qui est partiellement ou entièrement hydrogéné, ou phényle ou hétéroaryle, pris dans le groupe des restes pyridyle, pyrimidyle, pyridazinyle, pyrazinyle, thiényle, thiazolyle, oxazolyle, furyle, pyrrolyle, pyrazolyle et imiadazolyle,
chacun des restes alkyle, cycloalkyle, alcényle, alcynyle, hétérocyclyle, phényle et hétéroaryle cités en derniers sont, indépendamment les uns des autres, non substitués ou substitués par un ou plusieurs restes pris dans le groupe comportant des atomes d'halogènes, des groupes cyano, thio, nitro, hydroxy, alkyle en C₁-C₄, ce dernier seulement pour le cas de restes cycliques, haloalkyle en C₁-C₄, alkoxy en C₁-C₄, alcényloxy en C₂-C₄, alcynyloxy en C₂-C₄, haloalkoxy en C₁-C₄, (alkyl en C₁-C₄)thio, (alcényle en C₂-C₄)-thio, (alcynyle en C₂-C₄)thio, cycloalkyle en C₅-C₆, cycloalkoxy en C₅-C₆, amino, mono-et di-(alkyl en C₁-C₄)amino, (alkoxy en C₁-C₆)-carbonyle, des restes de formules -SiR'₃, -O-NR'₂, -O-N=CR'₂, -N=CR'₂, où R' dans les formules précitées représentent, indépendamment les uns des autres, des atomes d'hydrogène, des groupes alkyle en C₁-C₂ ou phényle ou par paires une chaîne alkylène en C₂-C₅.

5. Agent phytoprotecteur selon l'une des revendications 1 à 4, **caractérisé en ce que** dans la formule (I)
R³, R⁴ représentent, indépendamment l'un de l'autre, des restes identiques ou différents de formule 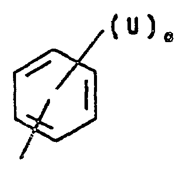
R représente un atome d'hydrogène, des groupes alkyle en C₁-C₈, cycloalkyle en C₄-C₇, alcényle en C₂-C₈ ou alcynyle en C₂-C₈, hétérocyclyle qui est un reste hétérocyclique de 3 à 7 chaînons dérivant du benzène, dans lequel au moins un groupe CH est remplacé par un atome de N et/ou au moins deux paires CH adjacents sont remplacés par des groupes NH, S et/ou O et qui est partiellement ou entièrement hydrogéné, ou phényle ou hétéroaryle pris dans le groupe des restes pyridyle, pyrimidyle, pyridazinyle, pyrazinyle, thiényle, thiazolyle, oxazolyle, furyle, pyrrolyle, pyrazolyle et imidazolyle,
chacun des restes alkyle, cycloalkyle, alcényle, alcynyle, hétérocyclyle, phényle et hétéroaryle cités en derniers sont, indépendamment les uns des autres, non substitués ou substitués par un ou plusieurs restes pris dans le groupe comportant des atomes d'halogènes, des groupes cyano, thio, nitro, hydroxy, alkyle en C₁-C₄, ce dernier seulement pour le cas de restes cycliques, haloalkyle en C₁-C₄, alkoxy en C₁-C₄, alcényloxy en C₂-C₄, alcynyloxy en C₂-C₄, haloalkoxy en C₁-C₄, (alkyl en C₁-C₄)thio, (alcényle en C₂-C₄)-thio, (alcynyle en C₂-C₄)thio, cycloalkyle en C₅-C₆, cycloalkoxy en C₅-C₆, amino, mono-et di-(alkyl en C₁-C₄)amino, (alkoxy en C₁-C₆)-carbonyle, des restes de formules -SiR'₃, -O-NR'₂, -O-N=CR'₂, -N=CR'₂, où R' dans les formules précitées représentent, indépendamment les uns des autres, des atomes d'hydrogène, des groupes alkyle en C₁-C₂ ou phényle ou par paires une chaîne alkylène en C₂-C₅, et
R^{T} représente un reste de formule -CO-R, -NR^{f}R^{g} ou -N=CR^{h}Rⁱ.

6. Agent phytoprotecteur selon l'une des revendications 1 à 5, **caractérisé en ce que** dans la formule (I)
R représente un atome d'hydrogène, des groupes alkyle en C₁-C₈, cycloalkyle en C₅-C₆, alcényle en C₂-C₈ ou alcynyle en C₂-C₈, les 4 derniers restes cités étant, indépendamment l'un de l'autre, non substitués ou substitués par un ou plusieurs restes pris dans le groupe comportant des atomes d'halogènes, des groupes cyano, nitro, alkoxy en C₁-C₄, alcényloxy en C₂-C₄, alcynyloxy en C₂-C₄, cycloalkyle en C₅-C₆, cycloalkoxy en C₅-C₆, mono-et di-(alkyl en C₁-C₄)amino, des restes de formules -SiR'₃, -O-N=CR'₂, -N=CR'₂, où R' dans les formules précitées représentent, indépendamment les uns des autres, des atomes d'hydrogène, des groupes alkyle en C₁-C₂ ou phényle ou par paires, une chaîne alkylène en C₂-C₅.

7. Agent phytoprotecteur selon l'une des revednications 1 à 6, **caractérisé en ce que** dans la formule (I)
R¹ représente un reste de formule 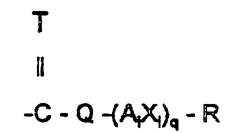 et
T représente un atome d'oxygène un groupe NR⁸,
Q représente un atome d'oxygène,
q est un entier de 0 à 4,
i est un chiffre qui, pour q inégal à 0, prend tous les entiers de 1 à q, q ayant la signification donnée ci-dessus,
Xᵢ représentent, indépendamment les uns des autres, des groupes O, S, NR⁹, N-(AᵢXᵢ)_{q}-R,
Aᵢ représentent, indépendamment les uns des autres, un groupe alkylène en C₁-C₄,
R⁸, R⁹ représentent, indépendamment l'un de l'autre, des atomes d'hydrogène, des groupes alkyle en C₁-C₄, alcényle en C₂-C₄, alcynyle en C₂-C₄ ou cycloalkyle en C₅-C₆.

8. Agent phytoprotecteur, **caractérisé en ce qu'**il contient au moins un pesticide ainsi que, en tant qu'antidote, au moins un composé de formule (I) ou ses sels selon l'une des revendications 1 à 7.

9. Composés de formule (I) et leurs sels tels que définis selon l'une des revendications 1 à 7, à l'exception de la 3-carbéthoxy-5-méthyl-5-vinylisoxazoline.

10. Procédé pour la préparation de composés de formule (I) ou leurs sels tels que définis selon la revendication 9, **caractérisé en ce qu'**on fait réagir un composé de formule (II)
R³R⁴C=CHR² (II)
où R², R³ et R⁴ sont définis comme pour le composé de formule (I), sur un oxyde de nitrile de formule (III)
⁽⁻⁾O-N=⁽⁺⁾C-R¹ (III)
où R¹ possède la signification donnée à la formule (I).

11. Procédé pour la protection de plantes de culture contre les effets secondaires phytotoxiques des substances actives phytoprotectrices (pesticides), **caractérisé en ce qu'**on applique une quantité efficace d'au moins un composé de formule (I) ou de ses sels selon l'une des revendications 1 à 7, avant ou en même temps que le pesticide concerné, sur les plantes, les grains ou la surface cultivable.

12. Utilisation de composés de formule (I) ou de leurs sels tels que définis selon l'une des revendications 1 à 7, en tant qu'antidote pour la protection de plantes de culture contre les effets secondaires phytotoxiques des substances actives phytoprotectrices (pesticides).
